# EUROPEAN PATENT SPECIFICATION

(11) **EP 4 468 970 B1**
(45) Date of publication and mention of the grant of the patent: **02.09.2026**
(21) Application number: 23704520.8
(22) Date of filing: 09.01.2023
(51) Int. Cl.: A61B 17/04, A61B 17/00, A61B 90/00, A61F 2/24

(54) **SIMULATION METHOD AND DEVICES FOR HEART VALVE REPAIR**
SIMULATIONSVERFAHREN UND VORRICHTUNGEN ZUR HERZKLAPPENREPARATUR
PROCÉDÉ DE SIMULATION ET DISPOSITIFS POUR LA RÉPARATION D'UNE VALVULE CARDIAQUE

(30) Priority: 27.01.2022 US 202263267226 P
(43) Date of publication of application: 04.12.2024
(73) Proprietor: Edwards Lifesciences Corporation, Irvine, CA 92614 (US)
(72) Inventor: CORTEZ, Felino, V., Jr., Bowie, MD 20715 (US); COURNANE, Stephen, Severn, MD 21144 (US); CHUNG, Nancy, Ling, Edgewater, MD 21037 (US); EPSTEIN, Stephen, Millersville, MD 21108 (US); ZANETTI, Luke, Anthony, Parkton, MD 21120 (US); HINGA (FORMERLY KEFFER), Ashley, Nicolette, Eldersburg, MD 217884 (US)
(74) Representative: Eisenführ Speiser
(86) International application number: PCT/US2023/010413
(87) International publication number: WO 2023/146748

(56) References cited:
- WO-A1-2017/059426
- WO-A1-2021/195460
- WO-A2-2019/236654
- US-A1- 2005 222 488
- US-A1- 2013 035 757

## Description

### BACKGROUND

The present disclosure generally relates to the field of cardiac valve repairs, and more particularly to minimally invasive cardiac valve repair operations. Cardiac valve abnormalities can adversely affect supply of oxygenated blood to the body. Insufficient supply of oxygen can produce any number of symptoms, adversely affecting quality of life. Cardiac valve repair procedures can be performed to treat the abnormalities and alleviate the symptoms.

US 2013/0035757 A1 describes a method of repairing a heart valve that provides intravascular access for repair of a heart valve through a ventricular transseptal approach. An external guide catheter is inserted through a vein of a patient into the right ventricle via the right atrium. An internal guide catheter is inserted through the external guide and provides access to the septum for a puncture tool to create an opening through the septum to the left ventricle. The internal guide is then advanced into the left ventricle and used to guide a deployment catheter that deploys a repair device onto the heart valve.

### SUMMARY

The present invention is defined in the appended claims. Additional aspects including methods relating to minimally invasive heart valve repair procedures are merely described for the purpose of understanding the functioning and possible applications of the invention. In some examples, described herein are systems and methods relating to minimally invasive heart valve repair procedures. In some instances, one or more portions of a heart valve annulus and/or mitral-annular curtain can be tethered to a ventricular wall. In some instances, a heart valve repair procedure can comprise tethering an anterior portion and a posterior portion of a heart valve annulus to one another. Tethering the heart valve annulus and/or mitral-annular curtain to the ventricular wall and/or tethering the anterior and posterior portions of the heart valve annulus to one another can reshape the heart valve orifice to facilitate desired heart valve leaflet coaptation.

Methods and structures disclosed herein for treating a patient also encompass analogous methods and structures performed on or placed on a simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (*e.g.,* thorax), a system (*e.g.,* cardiovascular system), an organ (*e.g.,* heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loud speakers, headphones, pressure transducers, temperature transducers, or using any combination of suitable technologies.

For purposes of summarizing the disclosure, certain aspects, advantages and novel features have been described herein. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the disclosure. In addition, various features of different disclosed examples can be combined to form additional examples, which are part of this disclosure. Throughout the drawings, reference numbers may be reused to indicate correspondence between reference elements. However, it should be understood that the use of similar reference numbers in connection with multiple drawings does not necessarily imply similarity between respective examples associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some examples or configurations.
Figure 1 is a cross-sectional view of a human heart and a tether deployed to couple a mitral valve leaflet and a tether deployed to couple a mitral valve annulus to a left ventricular wall, both tethers being anchored to the same location on the heart wall.
Figure 2 is a cross-sectional view of a human heart and a tether deployed to couple a mitral valve leaflet and a tether deployed to couple a mitral valve annulus to a left ventricular wall, the tethers being anchored to different location on the heart wall.
Figure 3 is a top-down view of a mitral valve and a plurality of tethers each comprising a distal portion coupled to respective portions of an annulus of the mitral valve.
Figures 4A, 4B and 4C show various parts of a process for deploying a tether to an annulus of a mitral valve using a tether deployment device.
Figure 5 is a side perspective view of the tether deployment device described with reference to the deployment process of Figure 4.
Figures 6A is a side view, Figure 6B is a side cross-sectional view, Figure 6C a top-down view, and Figure 6D a top-down perspective view, of an example of a distal portion of an elongate shaft that is part of a tether deployment device.
Figure 7 is a side cross-sectional view of another example of an elongate shaft of a tether deployment device.
Figure 8 is a side perspective view of an example of a portion of tether deployment device which includes an elongate shaft where a distal portion of the elongate shaft between a side opening and a distal end thereof is flexible.
Figure 9 is a side perspective view of an example of a portion of a tether deployment device that includes an atraumatic distal effector comprising an inflatable balloon.
Figure 10 is a top-down perspective view of a plurality of tethers coupling an anterior annulus portion of a mitral valve to a posterior annulus portions of the mitral valve.
Figure 11 shows deployment of a tether to an annulus of a mitral valve using a tether deployment device by accessing the annulus through a left atrium.
Figures 12A and 12B show a process of deploying a first tether and a second tether to a first position and a second position on an annulus, respectively.
Figures 13A and 13B show an example of a process to deploy a first tether to an anterior annulus portion and a second tether to a posterior annulus portion of a mitral valve.
Figures 14 shows an example of a first tether deployed to an anterior annulus portion and a second tether deployed to a posterior annulus portion, where the first tether and the second tether are coupled to one another over the anterior annulus portion.
Figures 15 shows an example of a first tether deployed to an anterior annulus portion and a second tether deployed to a posterior annulus portion, where the first tether and the second tether are coupled to one another over the posterior annulus portion.
Figure 16 shows a first tether deployed to an anterior annulus portion and a second tether deployed to a posterior annulus portion can be coupled to one another over a portion of the coaptation zone. A third tether deployed to another anterior annulus portion and a fourth tether deployed to another posterior annulus portion can be coupled to one another over another portion of the coaptation zone.
Figure 17 shows a first tether deployed to an anterior annulus portion and a second tether deployed to a posterior annulus portion can be coupled to one another over the anterior annulus portion. A third tether deployed to another anterior annulus portion and a fourth tether deployed to another posterior annulus portion can be coupled to one another over the other posterior annulus portion.
Figure 18 shows a first tether deployed to an anterior annulus portion and a second tether deployed to a posterior annulus portion can be coupled to one another over the posterior annulus portion. A third tether deployed to another anterior annulus portion and a fourth tether deployed to another posterior annulus portion can be coupled to one another over the other anterior annulus portion.
Figures 19A is a side view of the tether deployment device described with reference to the deployment process of Figures 11 and 12. Figure 19B is a more detailed side view, and Figure 19C is a side cross-sectional view, of the distal portion of the elongate shaft of the tether deployment device.
Figure 20 is a process flow diagram of an example of a process to deploy a tether.

### DETAILED DESCRIPTION

The headings provided herein are for convenience only and do not necessarily affect the scope or meaning of the claims.

In some examples, the present disclosure relates to systems and methods relating to minimally invasive heart valve repair procedures that can comprise tethering one or more portions of a heart valve annulus and/or mitral-annular curtain to a ventricular wall, and/or tethering an anterior portion and a posterior portion of a heart valve annulus to one another.

The scope of the claims that may arise herefrom is not limited by any of the particular examples described below. Furthermore, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/ element or anatomical structure to another device/ element or anatomical structure, it is understood that these terms are used herein for ease of description to describe the positional relationship between element(s)/ structures(s), as illustrated in the drawings. It should be understood that spatially relative terms are intended to encompass different orientations of the element(s)/ structures(s), in use or operation, in addition to the orientations depicted in the drawings. For example, an element/ structure described as "above" another element/ structure may represent a position that is below or beside such other element/ structure with respect to alternate orientations of the subject patient or element/ structure, and vice-versa.

Heart valve malfunction can occur due to valve regurgitation. Valve regurgitation occurs when a valve does not close properly. For example, regurgitation can occur due to improper coaptation of the valve leaflets. Improper coaptation of valve leaflets can be caused by one or more of dilation of the valve annulus, prolapse of one or more of the valve leaflets, and restriction in the motion of one or more leaflets. A dilated annulus can increase an area of the heart valve orifice such that the leaflets do not have sufficient surface area to cover the enlarged orifice. Leaflet prolapse can involve one or more leaflets having a segment above the plane of the annulus. Restriction in motion of one or more leaflets can be caused by one or more leaflets being constrained to below the plane of the annulus. Conditions such as mitral valve regurgitation may result in difficulty in pumping blood from the left atrium to the left ventricle, contributing to elevated pressure in the left atrium. Mitral valve regurgitation can result in blood flow leakage back into the left atrium from the left ventricle when the left ventricle contracts. Dysfunction of the mitral valve can contribute to elevated left atrial pressure. Elevated left atrial pressure may lead to left atrial enlargement, producing symptoms such as shortness of breath during exertion, fatigue, chest pain, fainting, abnormal heartbeat, and swelling of the legs and feet.

Described herein are systems and methods relating to repair of a heart valve, including a mitral valve. One or more of the heart valve repair procedures described herein can be performed as a part of a beating heart procedure, including a minimally invasive beating heart procedure. In some instances, a process for repairing a heart valve can comprise tethering one or more portions of a heart valve annulus and/or mitral-annular curtain to a ventricular wall. For example, distal portions of one or more tethers can be deployed to respective positions on a posterior portion of the heart valve annulus. Proximal portions of the one or more tethers can be anchored to the ventricular wall. The proximal portions can be anchored to an anterior portion of the ventricular wall. In some instances, the proximal portions can be anchored to a portion of the ventricular wall lateral of the left anterior descending artery (LAD). Tethering a posterior portion of the annulus to an anterior portion of the ventricular wall can provide force vectors that are anteriorly and basally directed to pull the posterior portion of the annulus down into the left ventricle and towards the anterior portion of the annulus. Pulling the posterior portion of the annulus down into the left ventricle and towards the anterior portion of the annulus can reduce an orifice size of the heart valve and/or reshape the orifice, thereby improving coaptation of the heart valve leaflets.

In some instances, a process for repairing a heart valve can comprise tethering an anterior portion and a posterior portion of a heart valve annulus to one another. For example, distal portions of one or more tethers can be deployed to respective positions on the anterior portion and distal portions of one or more tethers can be deployed to respective positions on the posterior portion. A proximal portion of a tether deployed to the anterior portion can be coupled to a proximal portion of a tether deployed to the posterior portion. In some instances, tethers coupled to opposing portions of the annulus can be coupled to one another. For example, each of one or more tethers deployed to respective positions on the anterior portion of the annulus can be coupled to a respective opposing position on the posterior portion of the annulus. Coupling tethers deployed to anterior portions of the annulus to tethers deployed to posterior portions of the annulus can facilitate reshaping of the annulus, and/or reducing a size of the heart valve orifice, thereby improving coaptation of the heart valve leaflets.

One or more processes described herein can comprise an annuloplasty procedure configured to modify the annulus, to thereby return the valve to a functional geometry, and alleviate mitral valve regurgitation. Modifying the heart valve orifice as described herein can be performed without cardiopulmonary bypass, cardiac arrest, and/or a large incision in the chest wall and heart, as required by typical annuloplasty procedures. Deploying tethers to the annulus can prevent or reduce recurrent annular dilatation, and/or provide improved repair security and/or durability, such as compared to deploying tethers to a portion of a heart valve leaflet. In some instances, use of tethers deployed to the annulus can be used for anatomies that are not suited for tethers deployed to a heart valve leaflet portion. The heart valve anatomy of some patients may not have a tissue-to-gap ratio (*e.g.,* a ratio of a length of a posterior leaflet to a gap length) desirable for deploying one or more tethers to a heart valve leaflet portion. For example, a tissue-to-gap ratio of about 2:1 or greater may be desirable for tethering a heart valve leaflet portion.

One or more processes for repairing a heart valve as described herein can be performed using a tether deployment device. The tether deployment device can comprise an elongate shaft comprising a lumen extending from a proximal end to a side opening on a distal portion of the elongate shaft. An atraumatic distal effector can be associated with the distal portion of the elongate shaft distal of the side opening. The tether deployment device can comprise a tether deployment assembly configured to be at least partially received in the lumen. The elongate shaft can comprise an inner wall defining the lumen. The inner wall can comprise a longitudinal wall portion extending from the proximal end of the elongate shaft to position proximal of the side opening. A curved wall portion can extend from the longitudinal wall portion to a distal end of the side opening to provide a curved path configured to guide an exit trajectory of a portion of the tether deployment assembly through the side opening. For example, the portion of the tether deployment assembly can slide and/or glide over and/or along at least a portion of the curved wall portion as it is advanced to the side opening, the curvature of the curved wall portion thereby guiding the exit of the tether deployment assembly. The tether deployment assembly can comprise a tether configured to be deployed to a target tissue.

In some instances, a method of deploying a tether to a heart tissue can comprise advancing a distal portion of the elongate shaft into a heart chamber. A distally oriented surface of the atraumatic distal effector can be positioned against a first tissue area adjacent to a target tissue area, while the side opening of the elongate shaft is oriented toward the target tissue area. A distal portion of the tether deployment assembly can be advanced through the side opening of the shaft. A portion of at least one tether can be deployed to the target tissue area using the tether deployment assembly while the distally oriented surface of the atraumatic distal effector is against the first heart tissue area.

In some instances, the distal portion of the elongate shaft can be advanced into a heart ventricle. The distally oriented surface of the atraumatic distal effector can be positioned against a portion of heart tissue adjacent to a target portion of the heart annulus oriented toward the heart ventricle. The distal portion of the tether deployment assembly can be advanced through the side opening of the shaft to deploy a distal portion of a tether to a portion of the heart annulus, including a posterior portion. A proximal portion of the tether can be anchored to a ventricular wall portion.

In some instances, the distal portion of the elongate shaft can be advanced into a heart atrium. The distally oriented surface of the atraumatic distal effector can be positioned against heart tissue adjacent to a portion of a heart annulus oriented toward the heart atrium. The distal portion of the tether deployment assembly can be advanced through the side opening of the shaft to deploy a distal portion of a first tether to an anterior portion of the heart annulus. One or more of these steps can be repeated to deploy a distal portion of a second tether to a posterior portion of the heart annulus. The first tether and the second tether can be coupled to one another over an atrium oriented surface portions of the heart valve. For example, proximal portions of the first and second tether can be coupled to one another over the atrium oriented surface.

The methods, operations, steps, *etc.* described herein can be performed on a living animal or on a non-living cadaver, cadaver heart, simulator (*e*.*g*., with the body parts, tissue, *etc.* being simulated), *etc.* For example, methods for treating a patient include methods for simulating the treatment on a simulated patient or anthropogenic ghost, which can include any combination of physical and virtual elements. Examples of physical elements include human or animal cadavers; any portions thereof, including organ systems, whole organs, or tissue; and manufactured elements, which can simulate the appearance, texture, resistance, or other characteristic. Virtual elements can include visual elements provided on a screen, or projected on a surface or volume, including virtual reality and augmented reality implementations. Virtual elements can also simulate other sensory stimuli, including sound, feel, and/or odor.

Any of the various systems, devices, apparatuses, etc. in this disclosure can be sterilized (*e*.*g*., with heat, radiation, ethylene oxide, hydrogen peroxide, etc.) to ensure they are safe for use with patients, and the methods herein can comprise sterilization of the associated system, device, apparatus, etc. (*e.g.,* with heat, radiation, ethylene oxide, hydrogen peroxide, etc.).

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

The term "suture" is used herein according to its plain and ordinary meaning and may refer to any elongate cord strip, strand, line, rope, wire, filament, tie, string, ribbon, strap, or portion thereof, or other type/form of material used in medical procedures (*e.g.,* ePTFE suture (*e*.*g*., GORE-TEX^{®} suture, W.L. Gore, Newark, Delaware), polyester suture (*e.g.,* ETHIBOND suture, Johnson & Johnson), UHMWPE suture (*e.g.,* FORCE FIBER suture, Teleflex), etc.). Furthermore, instances of the present disclosure may be implemented in connection with non-surgical and/or non-biological suture/line tensioning. With respect to the present disclosure, one having ordinary skill in the art will understand that a wire or other similar material may be used in place of a suture. Furthermore, in some contexts herein, the terms "cord" and "suture" may be used substantially interchangeably. In addition, use of the singular form of any of the suture-related terms listed above, including the terms "suture" and "cord," may be used to refer to a single suture/cord, or to a portion thereof. For example, where a suture knot or anchor is deployed on a distal side of a tissue portion, and where two suture portions extend from the knot/anchor on a proximal side of the tissue, either of the suture portions may be referred to as a "suture" or a "cord," regardless of whether both portions are part of a unitary suture or cord.

Although the target tissue is described primarily herein as comprising mitral valve heart tissue, it will be understood that the target tissue can comprise other heart valve tissue, including the tricuspid valve.

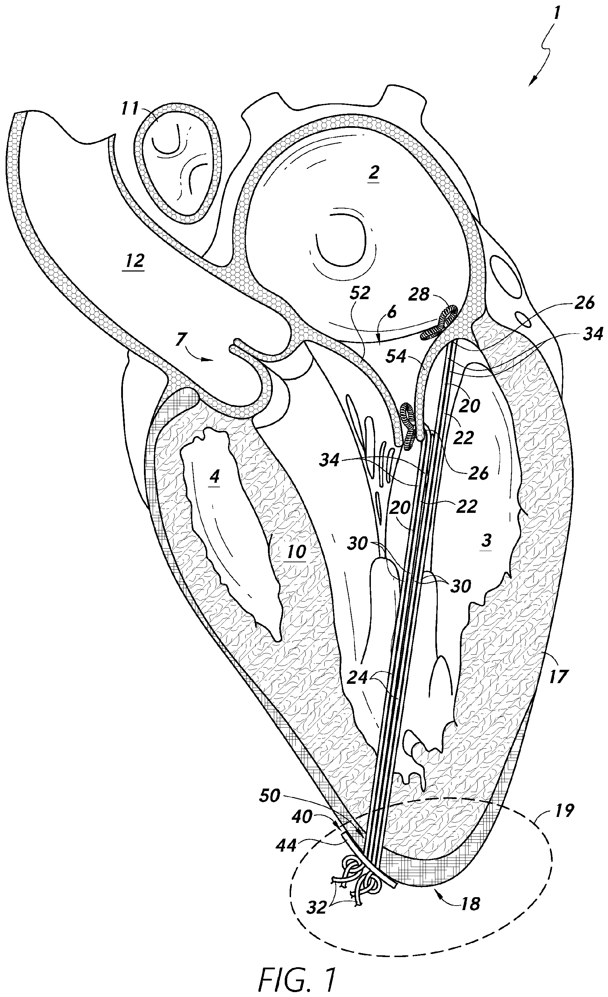

Figure 1 shows a cross-sectional view of a human heart. The heart 1 includes four chambers, namely the left atrium 2, the left ventricle 3, the right ventricle 4, and the right atrium. A wall of muscle, referred to as the septum 10, separates the left atrium 2 and right atrium, and the left ventricle 3 and right ventricle 4. Blood flow through the heart 1 is at least partially controlled by four valves, the mitral valve 6, aortic valve 7, tricuspid valve, and pulmonary valve. The mitral valve 6 separates the left atrium 2 and the left ventricle 3 and controls blood flow therebetween. The aortic valve 7 separates and controls blood flow between the left ventricle 3 and the aorta 12. The tricuspid valve separates the right atrium and the right ventricle 4 and controls blood flow therebetween. The pulmonary valve separates the right ventricle 4 and the pulmonary trunk or artery 11, controlling blood flow therebetween.

In a healthy heart, the heart valves can properly open and close in response to a pressure gradient present during various stages of the cardiac cycle (*e.g.,* relaxation and contraction) to at least partially control the flow of blood to a respective region of the heart and/or to blood vessels. Deoxygenated blood arriving from the rest of the body generally flows into the right side of the heart for transport to the lungs, and oxygenated blood from the lungs generally flows into the left side of the heart for transport to the rest of the body. During ventricular diastole, deoxygenated blood arrive in the right atrium from the inferior vena cava and superior vena cava to flow into the right ventricle 4, and oxygenated blood arrive in the left atrium 2 from the pulmonary veins to flow into the left ventricle 3. During ventricular systole, deoxygenated blood from the right ventricle 4 can flow into the pulmonary trunk 11 for transport to the lungs (*e.g.,* via the left and right pulmonary arteries), and oxygenated blood can flow from the left ventricle 3 to the aorta 12 for transport to the rest of the body.

As described herein, valve regurgitation occurs when a valve does not close properly. Heart valve repair performed to improve or restore valve function can comprise deploying one or more tethers to couple a heart valve leaflet and/or heart valve annulus to the heart wall to improve coaptation of the heart valve leaflets. For example, one or more tethers can be deployed to couple both a mitral valve leaflet and a portion of the mitral valve annulus to the left ventricular wall. Referring again to Figure 1, respective tethers 20 are shown coupling a leaflet of the mitral valve 6 and an annulus of the mitral valve 6 to the heart wall 17. A cross section is shown of an anterior leaflet 52 and a posterior leaflet 54 of the mitral valve 6. In some instances, more than one tether 20 can be deployed to each of a leaflet, including the posterior leaflet 54, such as a mid-segment of the posterior leaflet 54, and a portion of the annulus of the mitral valve 6, including a posterior portion of the annulus. In the alternative or in combination, one or more tethers 20 can be deployed to respective portions of a mitral-annular curtain. Each tether 20 can comprise a respective proximal portion 24 comprising an elongate portion 22, and a respective distal portion 26. The distal portion 26 of each tether 20 can be coupled to a respective position on the leaflet or annulus of the mitral valve 6. In some examples, each distal portion 26 can be associated with a respective anchor 28 to facilitate securing the tether 20 to the leaflet or the annulus. An anchor 28 can be positioned at least partially over an upper surface of the leaflet or at least partially over an upper surface of the annulus. For example, one or more anchors 28 can be positioned over each of an atrial facing surface of the posterior mitral valve leaflet 54 and posterior portion of the mitral valve annulus. In some instances, the anchor 28 can be a knot formed using a portion of the tether 20, such as the distal portion 26 of the tether 20. For example, the anchor 28 can be integral with the remainder of the tether 20, such as the proximal portion 24 of the tether 20. In some instances, the tether 20 can comprise a suture. In some instances, the anchor 28 can be formed using the same or similar material as the suture. The anchor 28 can comprise a suture knot, including a bulky suture knot. In some instances, the suture knot, such as the bulky suture knot, can be formed using a distal portion of the suture and be integral with the remainder of the suture. These tethers can be made from a variety of materials. One or more of these tethers can comprise for example expanded polytetrafluoroethylene (ePTFE). For example, each of the tethers 20 can be an ePTFE suture. In some instances, the tethers 20 can comprise polybutilate-coated polyester sutures. In some instances, the tethers 20 can comprise a malleable stainless steel, a shape memory material and/or a superelastic alloy.

The tethers 20 coupled to the posterior leaflet 54 and the posterior portion of the annulus can be tensioned anteriorly and downward into the left ventricle 3. The tethers 20 can couple the posterior leaflet 54 and annulus to a left ventricular portion of the heart wall 17. The proximal portion 24 of a tether 20 can be configured to be coupled to the heart wall 17, including an anterior portion of the heart wall 17. The proximal portion 24 of a tether 20 can be coupled to a portion of the heart wall 17 in which an opening 50 is formed to access the mitral valve 6. In some instances, a transapical approach can be used to gain access into the heart 1. For example, mitral valve repair procedures can include accessing the mitral valve 6 from within the left ventricle 3, where entry into the left ventricle 3 can be achieved through the opening 50 formed in the left ventricular wall in the apical region 19 of the heart 1. The heart wall 17 can be punctured in the apical region 19, such as in the apical region 19 on the left ventricular wall, offset from the apex 18, to form the opening 50. In some instances, the opening 50 can be formed in an anterior portion of the heart wall 17, including an anterior portion in the apical region 19. In some instances, the opening 50 can be formed in a region of the heart wall 17 that is lateral of the left anterior descending artery (LAD).

The apical region 19 is schematically shown in Figure 1 as the area within the dashed circle. As used herein, the "apical region" can include the inferior tip of the heart 1. The inferior tip is referred to herein as the apex 18 of the heart 1 and is generally located on the midclavicular line, in the fifth intercostal space. The apex 18 can be considered part of the greater apical region 19. Generally, the apical region 19 of the heart is a bottom region of the heart that is within the left or right ventricular region but is distal to the mitral 6 and tricuspid 8 valves and toward the tip of the heart 1. More specifically, the apical region 19 may be considered to be within about 20 centimeters (cm) to the right or to the left of the median axis of the heart 1.

The proximal portion 24 of a tether 20 can be anchored to a portion of the heart wall 17, including a portion of the heart wall 17 at or proximate to the apex 18 of the heart 1, such as in the apical region 19. In some instances, the proximal portion 24 of a tether 20 can be anchored to an anterior portion of the heart wall 17 in the apical region. In some instances, the proximal portion 24 of a tether 20 can be anchored to an anterior portion of the heart wall 17 lateral of the left anterior descending artery (LAD). A portion of an elongate portion 22 can extend at least partially through the heart wall 17 such that a proximal portion 24 of each tether 20 can be anchored at a position adjacent to an externally facing surface of the pericardium, epicardium or myocardium. In some instances, the proximal portion 24 of a tether 20 can be coupled to a surgical pad 40 positioned over, including on and in contact with, the pericardium, epicardium or myocardium, to facilitate anchoring the proximal portions 24 to the target location within the apical region 19 and maintain desired tension in the tethers 20. For example, the proximal portions 24 of the tethers 20 can be extended through the opening 50 in the heart wall 17, and through the surgical pad 40, to secure the tethers 20 the surgical pad 40. In some instances, anchoring the proximal portions 24 of the tethers 20 coupled to the posterior portion of the annulus to an anterior portion of the left ventricular wall in the apical region lateral to the left anterior descending artery (LAD) can provide force vectors that are anteriorly and basally directed pulling the posterior portion of the annulus down into the left ventricle and towards the anterior portion of the annulus. Pulling the posterior portions of the annulus downward and towards the anterior portion of the annulus can desirably reshape the shape of the heart valve orifice to facilitate leaflet coaptation.

In some cases, each elongate portion 22 can comprise a pair of tether tails 30. For example, each tether tail 30 can comprise a proximal portion 32 and a distal portion 34. Each of the distal portions 34 of the tether tails 30 can be associated with a corresponding suture knot 28. The proximal portions 32 can extend through the heart wall 17 and the surgical pad 40 such that the proximal portions 32 can be secured to the surgical pad 40, anchoring the tethers 20 to the heart wall 17. Figure 1 shows the tethers 20 all coupled to one location on the heart wall 17. The proximal portion 24 of a tether 20 can be anchored to a portion of the heart wall 17, including a portion of the heart wall 17 at or proximate to the apex 18 of the heart 1, such as in the apical region 19. In some instances, the proximal portion 24 of a tether 20 can be anchored to an anterior portion of the heart wall 17 in the apical region. In some instances, the proximal portion 24 of a tether 20 can be anchored to an anterior portion of the heart wall 17 lateral of the left anterior descending artery (LAD).

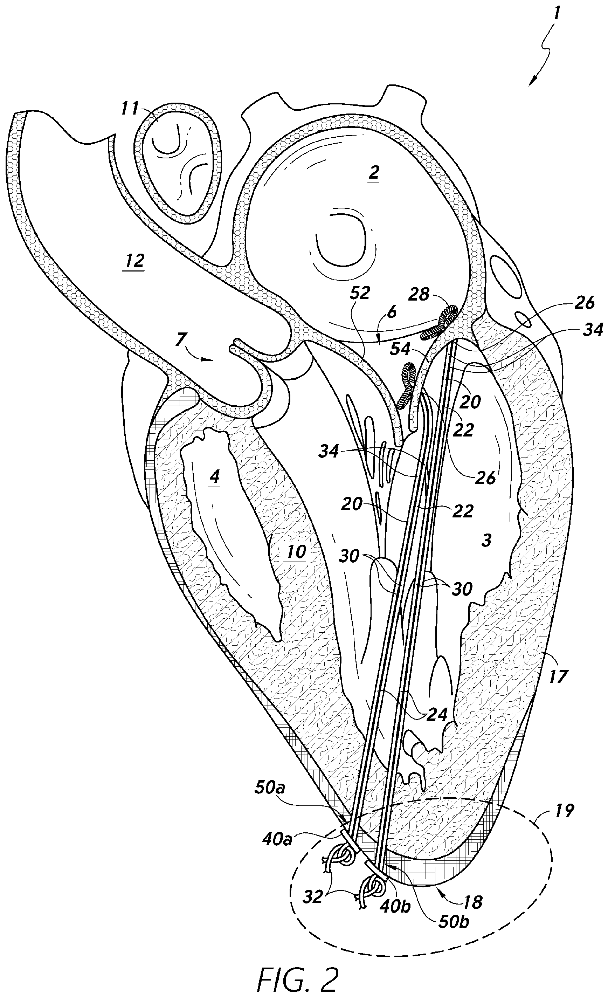

In an alternative example, as shown in Figure 2, the tethers 20 coupled to the heart valve leaflet and the annulus can be anchored to two different locations on the heart wall 17. For example, the distal portion 26 of each tether 20 can be coupled to a respective position on the leaflet or annulus of the mitral valve 6. Tethers 20 coupled to the heart valve leaflets can be anchored to a first position on the heart wall 17. Tethers 20 coupled to the annulus can be anchored to a second position on the heart wall 17. The proximal portions 24 of tethers 20 coupled to the heart valve leaflets can be secured to a first surgical pad 40a positioned over a first opening 50a formed at the first position in the heart wall 17. The proximal portions 24 of tethers 20 coupled to the annulus can be secured to a second surgical pad 40b positioned over a second opening 50b formed at the second position in the heart wall 17. The first and second positions can be selected based on desired force vectors formed by the tethers 20. In some instances, both the first and second positions can be on a portion of the heart wall 17 in the apical region 19, including an anterior portion of the heart wall 17 in the apical region. In some instances, the first and second positions can be on an anterior portion of the heart wall 17 lateral of the left anterior descending artery (LAD), including an anterior portion of the heart wall 17 lateral of the left anterior descending artery (LAD) in the apical region 19.

Coupling the leaflet and annulus to the heart wall 17 can facilitate reshaping of the mitral valve 6, such as to reduce or eliminate leaflet prolapse and/or reducing a size of the valve orifice. The tethers 20 can serve to improve coaptation of the mitral valve leaflets. Deploying tethers to the annulus can prevent or reduce recurrent annular dilatation, and/or provide improved repair security and/or durability, such as compared to deploying tethers to a portion of a heart valve leaflet. Tethering the annulus to the heart wall can modify the shape of annulus, facilitating return of the valve to a functional geometry. Use of tethers coupled to the annulus in combination with tethers deployed to the leaflets can decrease tension on the tethers deployed to the leaflets, thereby facilitating improved repair durability.

Tension in the tethers coupled to the annulus can be adjusted based at least in part on a desired positioning of the annulus throughout the cardiac cycle, such as to increase the amount of leaflet tissue available for coaptation. In some instances, tensioning of the tethers coupled to the annulus can be adjusted to prevent or reduce movement of the annulus in a posterior direction during systole. In some instances, one or more heart valve repair procedures as described herein can be performed while the heart is beating. Adjusting the tension of tethers while the heart is beating can advantageously allow the operator, such as a surgeon, to precisely titrate the position of the annulus in real time. Tethers coupled to the annulus and/or leaflet can be titrated individually or in groups, such as tethers to the annulus adjusted as a group or tethers adjusted to the leaflet adjusted as a group, providing greater flexibility in adjusting the coaptation of the leaflets, thereby enabling treating various configurations of diseased mitral valves.

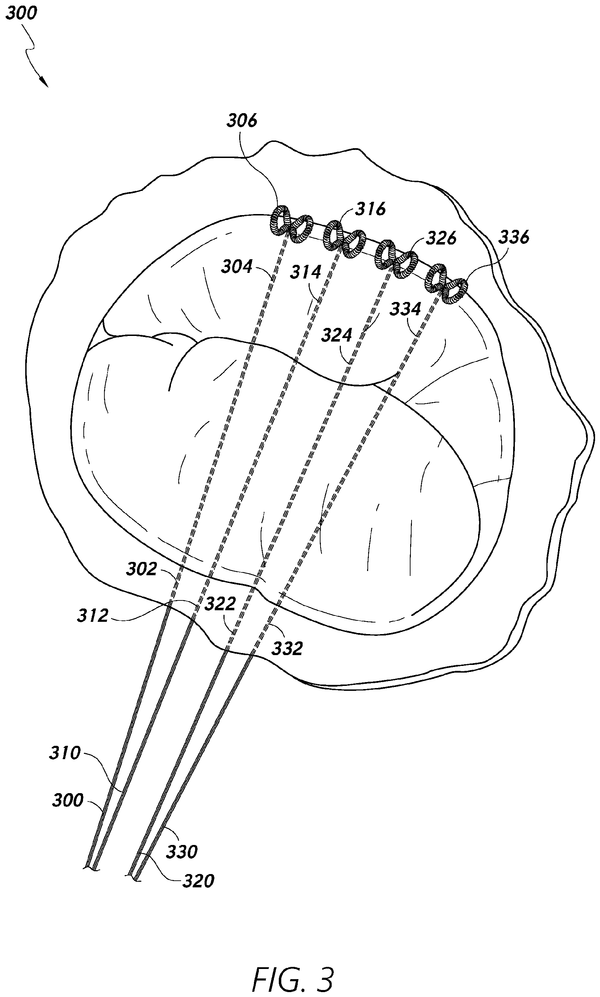

Figure 3 is a top-down view of a mitral valve and a plurality of tethers 300, 310, 320, 330 each comprising a distal portion 304, 314, 324, 334 coupled to respective portions of an annulus of the mitral valve. Respective distal portions 304, 314, 324, 334 of a first tether 300, second tether 310, third tether 320, and fourth tether 330 can be coupled to a posterior portion of the annulus. The distal portions 304, 314, 324, 334 can each comprise an anchor 306, 316, 326, 336. The anchors 306, 316, 326, 336 can be positioned at a first, second, third and fourth position, respectively, over a surface of the annulus oriented toward a left atrium. The first, second, third and fourth positions can be spaced from one another. In some instances, the first, second, third and fourth positions can be evenly spaced along a portion of the annulus, including on a posterior portion of the annulus. In some instances, the distal portions 304, 314, 324, 334 of the plurality of tethers 300, 310, 320, 330 can be coupled to respective portions of the annulus adjacent to the posteromiddle scallop of the posterior mitral valve leaflet.

Each tether 300, 310, 320, 330 can extend from the atrium-oriented surface through the annulus to a ventricle-oriented surface facing the left ventricle. Each tether 300, 310, 320, 330 can extend proximally from the annulus to an anchor location on a portion of the left ventricular wall. For example, a portion of each of the tethers 300, 310, 320, 330, including a proximal portion 302, 312, 322, 332, can be within the left ventricle. Respective proximal portions 302, 312, 322, 332 of each of the tethers 300, 310, 320, 330 can be coupled to a ventricular wall portion, such as an anterior portion of the ventricular wall. For example, proximal portions 302, 312, 322, 332 of each of the tethers 300, 310, 320, 330 can be anchored to an anterior portion of the ventricular wall. The plurality of tethers 300, 310, 320, 330 can be anchored to the same location on the ventricular wall, such as the location through which a tether deployment device is inserted to deploy the tethers 300, 310, 320, 330.

Figure 3 shows four tethers 300, 310, 320, 330 coupled to the annulus. It will be understood that more or fewer tethers can be applicable. The anchors 306, 316, 326, 336 can be bulky suture knots. The locations at which the tethers can be coupled to the annulus and/or number of tethers can be selected based on anatomical needs of the patient. The tethers can comprise one or more features of other tethers described herein. In some instances, each tether can comprise an elongate portion comprising a pair of tether tails. A proximal portion of the elongate portion can be configured to be coupled to the ventricular wall. A distal portion of the elongate portion can be associated with an anchor configured to be deployed to the mitral valve. For example, a bulky suture knot can be coupled to the distal portion of the elongate portion.

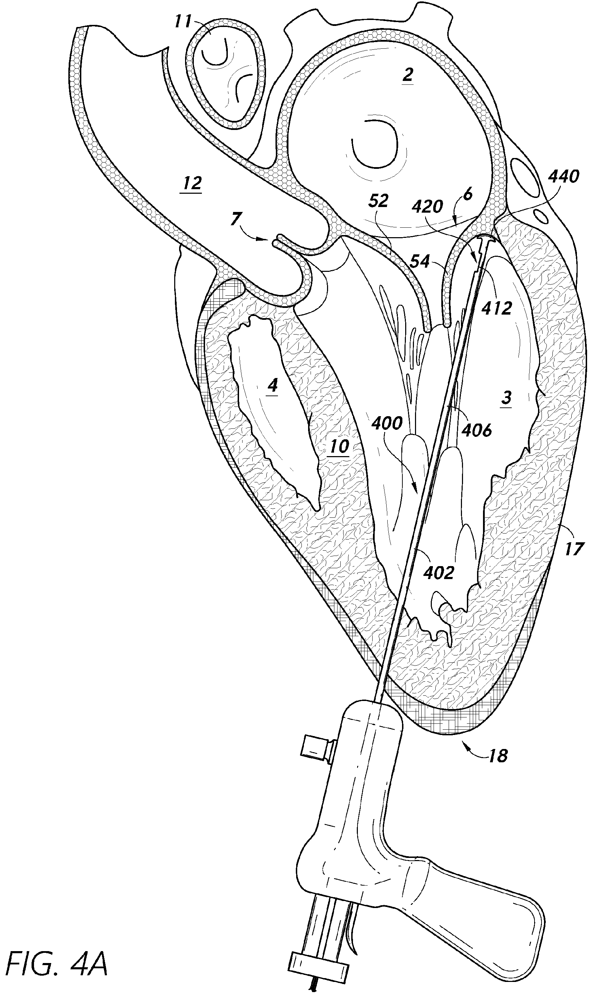

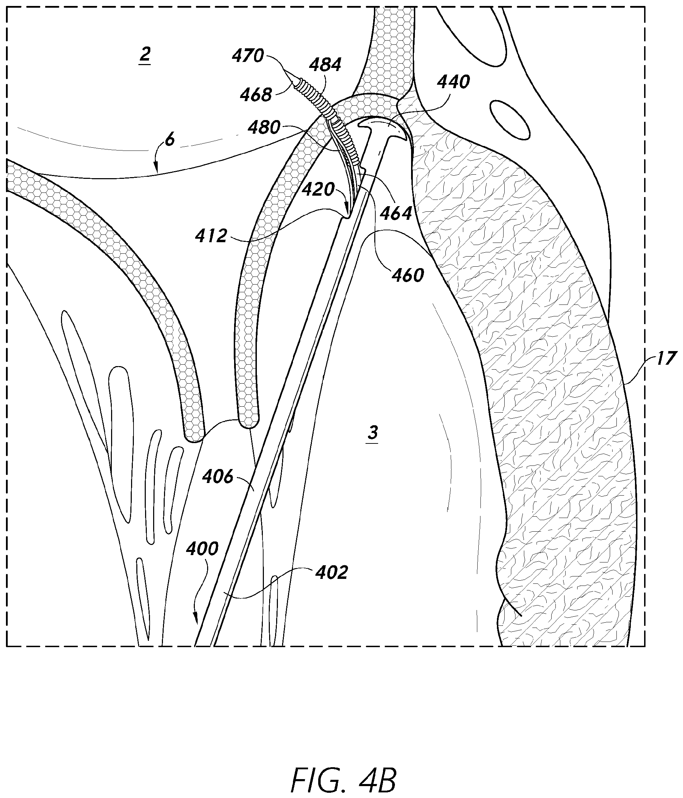

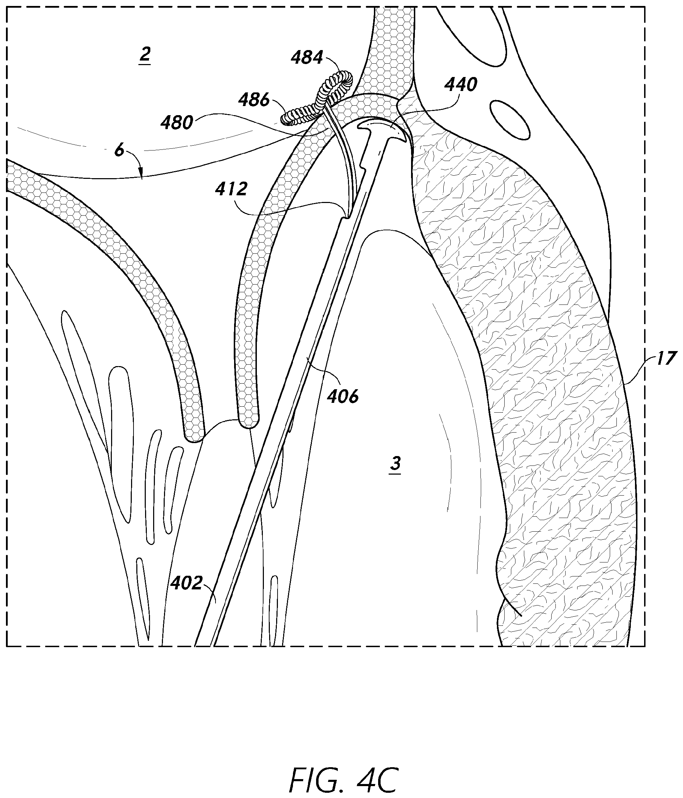

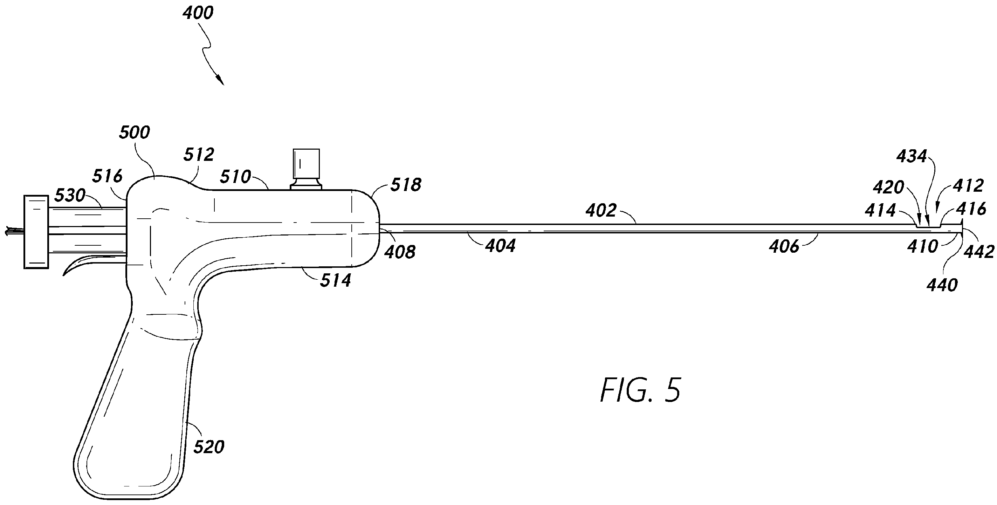

Figures 4A, 4B and 4C show various parts of a process for deploying a tether to an annulus of a mitral valve using a tether deployment device 400. As described in further detail herein, the tether deployment device 400 can comprise an elongate shaft 402. The elongate shaft 402 can comprise a lumen 420 extending from a proximal end 408 (as shown in Figure 5) to a side opening 412 on a distal portion 406 thereof. A tether deployment assembly 460 can be configured to be at least partially received within the lumen 420. The tether deployment assembly 460 can have a tether 480 comprising a distal portion 484 configured to be deployed through the side opening 412 to a target tissue area. Referring to Figure 4A, at least a portion of the elongate shaft 402 can be advanced into a left ventricle 3 through an opening formed on a heart wall 17 of the left ventricle 3. As described herein, the opening can be on an anterior portion of the left ventricular wall. In some instances, the opening can be on apical region of the ventricular wall, including on an anterior portion of the apical region. For example, a distal portion 406 of the elongate shaft 402 can be inserted through the opening formed in the left ventricular wall and advanced toward the mitral valve 6. The tether deployment device 400 can comprise an atraumatic distal effector 440 associated with the distal portion 406 of the elongate shaft 402, for example coupled to the distal portion 406 at a position distal of the side opening 412. The elongate shaft 402 can be advanced into the left ventricle 3 until the atraumatic distal effector 440 contacts heart tissue proximate to a target tissue area to which the distal portion 484 of the tether 480 can be deployed. In some instances, a portion of the atraumatic distal effector 440 can be positioned against a first tissue area that is adjacent to the target tissue area. In some instances, a portion of the atraumatic distal effector 440 can be positioned against tissue adjacent to the annulus and/or against a portion of the annulus. Advancement of the elongate shaft 402 into the left ventricle 3 and/or placement of the atraumatic distal effector 440 against the first tissue area can be guided using any number of imaging techniques, including for example transesophageal echocardiography (TEE), intracardiac echocardiography (ICE) and/or fluoroscopy.

In some instances, the target tissue area can be a portion of the annulus of the mitral valve 6. The target tissue area can be a posterior portion of the mitral valve annulus, including a posterior portion of the mitral valve annulus adjacent to the posteromiddle scallop of a posterior mitral valve leaflet 54. In some instances, the first tissue area can be a portion of heart tissue adjacent to the target portion of the mitral valve annulus. In some instances, the first tissue area can be a portion of tissue of a junction between the ventricular heart wall and the mitral valve annulus.

In some instances, the atraumatic distal effector 440 can contact the first tissue area while the side opening 412 is oriented toward the target portion of the mitral valve annulus. For example, the side opening 412 can be oriented toward a target tissue area on a posterior portion of a mitral valve annulus, while the atraumatic distal effector 440 is in contact with the junction between the heart wall and the mitral valve annulus. Contact between the atraumatic distal effector 440 and the first tissue area can facilitate stabilizing a position and/or orientation of the side opening 412 during deployment of the tether 480, thereby facilitating desired deployment of the tether 480.

Referring to Figure 4B, a distal portion 464 of the tether deployment assembly 460 can be advanced through the side opening 412 of the elongate shaft 402. An exit trajectory of the distal portion 464 from the elongate shaft 402 through the side opening 412 can be at an angle relative to the elongate shaft 402. The tether deployment assembly 460 can comprise a needle 470 at a distal end 468. The distal portion 464 can be advanced out of the side opening 412 such that the needle 470 at the distal end 468 can puncture and be advanced through the target tissue area. As shown in Figure 4B, the needle 470 can be advanced through a target tissue area that is adjacent to the first tissue area contacted by the atraumatic distal effector 440. The exit trajectory of the distal portion 464 of the tether deployment assembly 460 can be away from the heart wall 17, including an atrial and/or ventricular wall portion, to avoid accidental puncture of the heart wall. The needle 470 can puncture through the target tissue area from a first surface oriented toward the left ventricle 3 through to a second surface oriented toward the left atrium 2. The distal portion 464 of the tether deployment assembly 460 can comprise the distal portion 484 of the tether 480. A portion of the tether 480, including the distal portion 484 of the tether 480, is shown as being advanced out of the side opening 412 and through the target tissue area, as the distal portion 464 of the tether deployment assembly 460 is advanced out of the side opening 412. The distal portion 484 of the tether 480 can be deployed from the tether deployment assembly 460 to form an anchor over the second surface of the target tissue area. In some instances, the distal portion 484 can comprise a pre-formed tissue anchor sutureform that can be deployed to the target tissue area. For example, a bulky suture knot can be formed using the pre-formed tissue anchor sutureform.

Referring to Figure 4C, an anchor 486 is shown as being disposed over the second surface of the target tissue area oriented toward the left atrium 2. As described herein, the anchor 486 can be formed using the distal portion 484 of the tether 480. The tether deployment assembly 460 can be retracted after the anchor 486 is deployed, for example withdrawn back into the lumen 420 of the elongate shaft 402. The tether 480 can be extended out from through the side opening 412 as the elongate shaft 402 is retracted. For example, as the elongate shaft 402 is retracted, additional portions of the tether 480 can be extended out through the side opening 412. As the elongate shaft 402 is withdrawn from the left ventricle 3 through the opening formed on left ventricular wall, a portion of the tether 480 can remain extending between the annulus and the opening formed in the left ventricular wall. A proximal portion of the tether 480 can be anchored to the left ventricular heart wall, such as at the opening formed in the left ventricular heart wall through which the elongate shaft 402 is inserted.

Figure 5 is a side perspective view of the tether deployment device 400 described with reference to the deployment process of Figure 4. The tether deployment device 400 can comprise the elongate shaft 402 extending distally from a handle 500. For example, the proximal end 408 of the elongate shaft 402 can be coupled to the handle 500. The lumen 420 of the elongate shaft 402 can extend within the elongate shaft 402 from the proximal end 408 to the side opening 412 on the distal portion 406 of the elongate shaft 402. The tether deployment assembly 460 can be configured to be at least partially received within the lumen 420. The tether deployment assembly 460 (as shown in Figure 4) can comprise the tether 480 (as shown in Figure 4) for deployment to a target tissue area. The tether deployment assembly 460 can comprise a needle 470 at a distal end 468. Deployment of the tether 480 can comprise advancing the distal portion 464 of the tether deployment assembly 460 through the side opening 412 of the elongate shaft 402. The distal portion 464 can be advanced out of the side opening 412 such that the needle 470 at the distal end 468 can puncture and be advanced through the target tissue area to facilitate deployment of the tether 480 to the target tissue area.

An inner wall 430 (not shown) of the elongate shaft 402 can define the lumen 420. A longitudinal wall portion 432 of the inner wall 430 can define a longitudinal portion of the lumen 420. The longitudinal portion of the lumen 420 can extend along an axis parallel or substantially parallel to a longitudinal axis of the elongate shaft 402. In some instances, the longitudinal wall portion 432 can extend from the proximal end 408 of the elongate shaft 402 to the proximal end 414 of the side opening 412. For example, the longitudinal wall portion 432 can define the longitudinal portion of the lumen 420 that extends from the proximal end 408 of the elongate shaft 402 to the proximal end 414 of the side opening 412.

The inner wall 430 can have a curved wall portion 436 proximate and/or adjacent to the side opening 412. The curved wall portion 436 can be distal of the longitudinal wall portion 432. The curved wall portion 436 can define a curved path extending between the longitudinal portion of the lumen 420 and the side opening 412 and configured to guide the exit trajectory of the distal portion 464 of the tether deployment assembly 460 through the side opening 412. For example, a distal portion 434 of the inner wall 430 can comprise the curved wall portion 436. In some instances, the curved wall portion 436 can extend from the longitudinal wall portion 432 to the side opening 412. The curved wall portion 436 can define a curved path that extends from the longitudinal portion of the lumen 420 to the side opening 412.

In some instances, an opposing portion of the inner wall 430 opposite the side opening 412 can comprise the curved wall portion 436. In some instances, the curvature of the curved wall portion 436 can extend from a portion opposite the proximal end 414 of the side opening 412 to a distal end 416 of the side opening 412. At least a portion of tether deployment assembly 460 can contact the curved wall portion 436 as the portion is advanced out of the side opening 412 such that the curvature of the curved wall portion 436 can determine at least in part the angle the portion of the tether deployment assembly 460 forms with the longitudinal axis of the elongate shaft 402 after the portion is advanced through the side opening 412. For example, one or more portions of the tether deployment assembly 460, including the distal portion 464 of the tether deployment assembly 460, can slide and/or glide over and/or along the curved wall portion 436 as the distal portion 464 is advanced to the distal end of the lumen 420 and out of the side opening 412. In some instances, the tether 480, including the distal portion 484 of the tether 480, can slide and/or glide over and/or along the curved wall portion 436 as the distal portion 464 is advanced to the distal end of the lumen 420 and out of the side opening 412.

The atraumatic distal effector 440 can be coupled to at least a portion of the distal portion 406 of the elongate shaft 402. The atraumatic distal effector 440 can be disposed distally of the side opening 412. In some instances, the atraumatic distal effector 440 can be at a distal end 410 of the elongate shaft 402. In some instances, the atraumatic distal effector 440 can comprise a distally oriented planar or substantially planar surface 442 configured to engage with a first tissue area adjacent to the target tissue area, including distally oriented planar surfaces. The atraumatic distal effector 440 can comprise one or more distally oriented round planar or substantially planar surfaces, such as one or more circular or substantially circular planar surfaces. In some instances, the atraumatic distal effector 440 can comprise a disc configuration. The atraumatic distal effector 440 can comprise a flexible disc coupled to the distal end 410 of the elongate shaft 402. A distally oriented surface of the flexible disc can be configured to contact the first tissue area.

In some instances, the distally oriented surface of the atraumatic distal effector 440 can be perpendicularly or substantially perpendicularly oriented relative to the longitudinal axis of the elongate shaft 402. In some instances, the distally oriented surface of the disc can be perpendicular or substantially perpendicular to the longitudinal axis. For example, the disc, such as the flexible disc, can be perpendicularly or substantially perpendicularly oriented relative to the longitudinal axis of the elongate shaft 402.

Referring again to Figure 5, the tether deployment device 400 can comprise the handle 500. As described herein, the proximal end 408 of the elongate shaft 402 can be coupled to the handle 500. The handle 500 can comprise a longitudinal portion 510 comprising a longitudinal axis that is parallel or substantially parallel to the longitudinal of the elongate shaft 402. The longitudinal portion 510 can extend along the axis parallel or substantially parallel to the longitudinal of the elongate shaft 402. The handle 500 can comprise a lateral portion 520 extending laterally from the longitudinal portion 510. The lateral portion 520 can extend at an angle from the longitudinal portion 510, for example extending at an angle relative to the longitudinal axis of the elongate shaft 402. The lateral portion 520 can be configured to engage with an operator. For example, an operator can grasp onto the lateral portion 520 while manipulating the tether deployment device 400. In some instances, the lateral portion 520 can extend from a proximal portion 512 of the longitudinal portion 510.

The proximal portion 404 of the elongate shaft 402 may be coupled to a handle 500, including to the longitudinal portion 510 of the handle 500. For example, the proximal end 408 of the elongate shaft 402 may extend from a distal portion 514, including a distal end 518, of the longitudinal portion 510.

In some instances, the side opening 412 of the elongate shaft 402 can have an orientation opposite that of the lateral portion 520. For example, the lateral portion 520 can extend along a first direction relative to the longitudinal axis of the elongate shaft 402 and the side opening 412 can be on a portion of the elongate shaft 402 oriented toward a second direction opposite that of the first direction relative to the longitudinal axis of the elongate shaft 402. For example, the side opening 412 can be dorsally oriented on the elongate shaft 402.

A plunger 530 can be partially movably disposed within the handle 500, for example extending from a proximal end 516 of the longitudinal portion 510 of the handle 500. The tether deployment assembly 460 can be coupled to the plunger 530. The plunger 530 can be used to actuate or move the tether deployment assembly 460 during deployment of the tether 480. Alternatively or in combination, other means of actuating or moving the tether deployment assembly 460 can be used. In some instances, the tether deployment device 400 can comprise a trigger mechanism, such as a trigger mechanism at least partially housed within the handle 500, configured to actuate or move the tether deployment assembly 460. In some instances, the tether deployment device 400 can comprise a spring component, gear and rack, six bar linkage and/or electronic motor, such as at least partially housed within the handle 500, configured to actuate or move the tether deployment assembly 460.

The tether deployment device 400 can be used to deploy one or more other tethers described herein, including tethers described with reference to Figures 1, 2 and 3.

One or more anchors as described herein can formed using a number of different techniques. In some instances, an anchor can be formed using one or more methods as described in U.S. Patent No. 10,765,515, entitled "Distal Anchor Apparatus and Methods for Mitral Valve Repair," and issued on September 8, 2020. In some instances, an anchor can be formed using one or more methods as described in U.S. Patent No. 10,864,080, entitled "Distal Anchor Apparatus and Methods for Mitral Valve Repair," and issued on December 15, 2020.

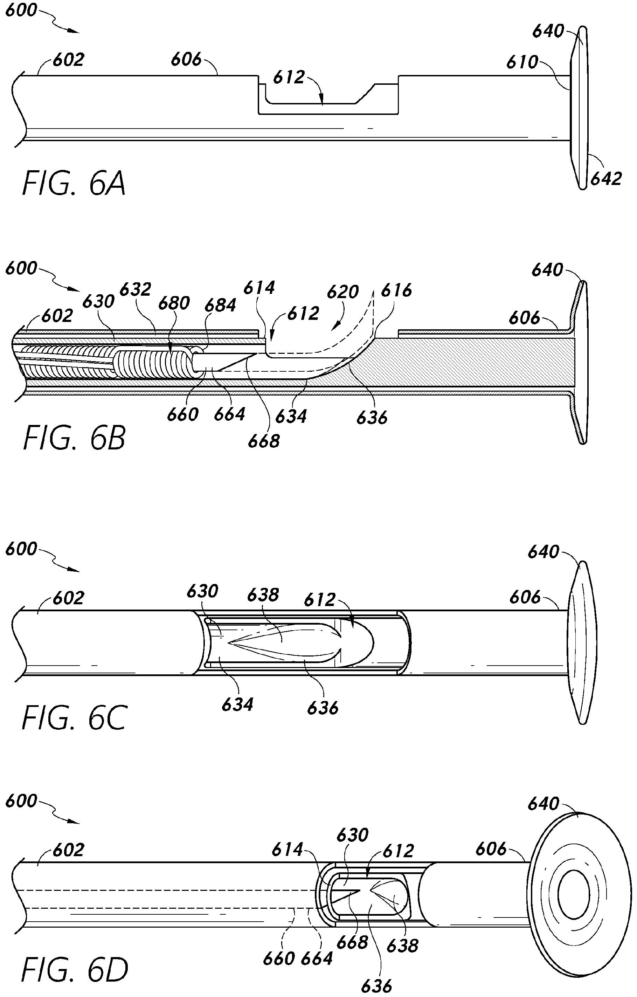

Figures 6A, 6B, 6C and 6D are various views of an example of a distal portion 606 of an elongate shaft 602 that is part of a tether deployment device 600. The remainder of the tether deployment device 600 is not shown for simplicity. Referring to Figure 6A, a side view of the distal portion 606 of the elongate shaft 602 is shown. The distal portion 606 can comprise a side opening 612 in communication with a lumen 620 extending within the elongate shaft 602 from a proximal end of the elongate shaft 602. As described in further detail herein, a portion of a tether deployment assembly 660 can be advanced through the side opening 612 to facilitate deployment of a tether 680 carried by the tether deployment assembly 660.

The tether deployment device 600 can include an atraumatic distal effector 640 coupled to a distal end 610 of the elongate shaft 602. For example, the atraumatic distal effector 640 can be at the distal end 610 of the elongate shaft 602. The atraumatic distal effector 640 can comprise one or more distally oriented planar or substantially planar surfaces configured to contact a first tissue area. In some instances, the atraumatic distal effector 640 can comprise a disc configuration. A distal surface 642 of the atraumatic distal effector 640 can be configured to contact a first tissue area adjacent to a target tissue area. For example, the atraumatic distal effector 640 can comprise a disc, including a circular disc, having a distally oriented surface configured to contact the first tissue area. The atraumatic distal effector 640 can comprise at least a portion that is flexible, such as to facilitate positioning of the atraumatic distal effector 640 against a desired tissue area while reducing or preventing damage to the tissue. In some instances, the disc can comprise at least a portion that is flexible. In some instances, the atraumatic distal effector 640 can comprise a flexible disc that has a distally oriented surface configured to contact the first tissue area.

In some instances, the atraumatic distal effector 640 can have an orientation that is perpendicular or substantially perpendicular to a longitudinal axis of the elongate shaft 602. The distal surface 642 of the atraumatic distal effector 640 can be perpendicularly oriented relative to the longitudinal axis of the elongate shaft 602. For example, the atraumatic distal effector 640 can comprise a disc coupled to the distal end 610 of the elongate shaft 602 and is perpendicularly or substantially perpendicularly oriented relative to the longitudinal axis of the elongate shaft 602.

Figure 6B is a side cross-sectional view of the distal portion 606 of the elongate shaft 602. The tether deployment assembly 660 is shown as being preloaded within the elongate shaft 602. As described herein, at least a portion of a tether deployment assembly 660 can be slidably received within the lumen 620. A portion, such as a distal portion 664 of the tether deployment assembly 660, can be advanced through the side opening 612 for deployment of the tether 680 carried by the tether deployment assembly 660. A distal portion 684 of the tether 680 can be deployed from the tether deployment assembly 660 to form an anchor at a target tissue area. For example, while the elongate shaft 602 is advanced to a target position, the deployment assembly 660 can be disposed within the lumen 620. A distal end 668 of the tether deployment assembly 660 can be disposed proximally of the side opening 612, including adjacent to a proximal end 614 of the side opening 612.

An inner wall 630 of the elongate shaft 602 defining the lumen 620 can comprise a longitudinal wall portion 632 defining a longitudinal portion of the lumen 620. The longitudinal portion of the lumen 620 can extend along an axis parallel or substantially parallel to a longitudinal axis of the elongate shaft 602. In some instances, the longitudinal wall portion 632 can extend from the proximal end of the elongate shaft 602 to the proximal end 614 of the side opening 612, defining a longitudinal portion of the lumen 620 extending from the proximal end of the elongate shaft 602 to the proximal end 614 of the side opening 612.

A distal portion 634 of the inner wall 630 can comprise a curved wall portion 636. The curved wall portion 636 can be distal of the longitudinal wall portion 632. The curved wall portion 636 can define a curved portion of the lumen 620 extending between the longitudinal portion of the lumen 620 and the side opening 612. The curved wall portion 636 can define a curved path for the tether deployment assembly 660 and configured to guide the exit trajectory of a portion of the tether deployment assembly 660 through the side opening 612. In some instances, the curved wall portion 636 can extend from the longitudinal wall portion 632 to the side opening 612 to define a curved path that extends from the longitudinal portion of the lumen 620 to the side opening 612. The curved wall portion 636 can comprise at least a portion of the inner wall 630 opposite the side opening 612. In some instances, the curvature of the curved wall portion 636 can extend from a portion of the inner wall 630 opposite the proximal end 614 of the side opening 612 to a portion of the inner wall 630 at, adjacent or proximate to, a distal end 616 of the side opening 612. In some instances, the curvature of the curved wall portion 636 can extend from a portion of the inner wall 630 that is opposite the side opening 612 and distal of the proximal end 614 of the side opening 612 to a portion of the inner wall 630 at, adjacent or proximate to, a distal end 616 of the side opening 612. In some instances, the curved wall portion 636 can define a curved path that forms a segment of an oval and/or circle. The degree of curvature of the curved path can depend at least in part on the desired exit trajectory of the tether deployment assembly 660.

At least a portion of the tether deployment assembly 660 can contact the curved wall portion 636 as the portion of the tether deployment assembly 660 is advanced out of the side opening 612. The curvature of the curved wall portion 636 can determine at least in part the angle the portion of the tether deployment assembly 660 disposed externally of the side opening 612 forms with the longitudinal axis of the elongate shaft 602. For example, at least a portion of the tether deployment assembly 660 can be flexible, including the distal portion 664 of the tether deployment assembly 660. One or more flexible portions of the tether deployment assembly 660 can slide and/or glide over and/or along the curved wall portion 636 as the one or more flexible portions is advanced to the distal end of the lumen 620 and out of the side opening 612. In some instances, one or more portions of the tether 680 carried by the tether deployment assembly 660 can slide and/or glide over and/or along the curved wall portion 636 as a portion of the tether deployment assembly 660 is deployed through the side opening 612.

Figure 6C is a top-down view of the distal portion 606 of the elongate shaft 602. As described herein, the distal portion 634 of the inner wall 630 can comprise the curved wall portion 636 configured to guide the exit trajectory of the portion of the tether deployment assembly 660 that is advanced through the side opening 612. In some instances, the distal portion 634 of the inner wall 630 can comprise a groove 638 configured to guide movement of the portion of the tether deployment assembly 660 through the lumen 620 as the portion of the tether deployment assembly 660 is advanced distally to the side opening 612. For example, the groove 638 can be on at least a portion of the inner wall 630 opposite the side opening 612. The groove 638 can have the same or similar orientation as the curvature of the curved wall portion 636 configured to guide the exit trajectory of the tether deployment assembly 660. In some instances, the curved wall portion 636 can define at least a portion of the groove 638. For example, the groove 638 can extend along a direction parallel or substantially parallel to the curvature of the curved wall portion 636 extending from the longitudinal wall portion 632 to the side opening 612. In some instances, the portion of the tether deployment assembly 660, such as a least a portion of the distal portion 664 of the tether deployment assembly 660, can be in contact with at least a portion of the groove 638 as the portion is advanced to the side opening 612. In some instances, the portion of the tether deployment assembly 660 can slide and/or glide over and/or along the groove 638 as the portion is advanced through the lumen 620 to the side opening 612. In some instances, a portion of the tether 680, such as the distal portion 684 of the tether 680, can be in contact with at least a portion of the groove 638 as the portion of the tether deployment assembly 660 is advanced to the side opening 612. In some instances, a portion of the tether 680, such as the distal portion 684 of the tether 680, can glide over and/or along the groove 638.

Figure 6D is a top-down perspective view of the distal portion 606 of the elongate shaft 602 and the side opening 612 on the distal portion 606. A portion of the groove 638 on the inner wall 630 opposite the side opening 612 is shown in the figure. The tether deployment assembly 660 is shown as being preloaded within the elongate shaft 602. The distal end 668 of the tether deployment assembly 660 can be disposed proximally of the side opening 612, including adjacent to the proximal end 614 of the side opening 612. A portion of the tether deployment assembly 660, including a portion of the tether 680, can slide and/or glide over and/or along the curved wall portion 636 and the groove 638 as it is advanced through the lumen 620 to the side opening 612.

In some instances, the tether deployment device 600 can comprise one or more other features of the tether deployment device 400 described with reference to Figure 5. For example, the elongate shaft 602 can comprise one or more other features of the elongate shaft 402. In some instances, the elongate shaft 602 can extend distally from a handle having the same or similar features as the handle 500 describe with reference to Figure 5.

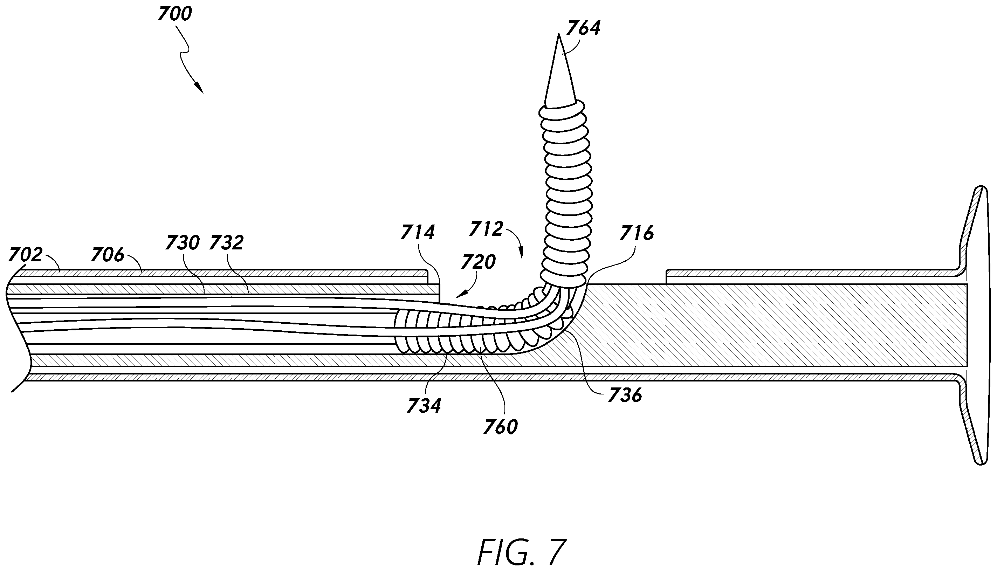

Figure 7 is a side cross-sectional view of another example of an elongate shaft 702 of a tether deployment device 700. The elongate shaft 702 can comprise an inner wall 730 that defines a lumen 720 extending from a proximal end of the elongate shaft 702 to a side opening 712 on a distal portion 706 of the elongate shaft 702. The inner wall 730 can comprise a longitudinal wall portion 732 defining a longitudinal portion of the lumen 720 that extends along an axis parallel or substantially parallel to a longitudinal axis of the elongate shaft 702. In some instances, the longitudinal wall portion 732 can extend from the proximal end of the elongate shaft 702 to the proximal end 714 of the side opening 712. A distal portion 734 of the inner wall 730, including at least a portion of the inner wall 730 opposite the side opening 712, can comprise a curved wall portion 736 distal of the longitudinal wall portion 732. For example, the curved wall portion 736 can extend from the longitudinal wall portion 732 to a distal end 716 of the side opening 712. The curved wall portion 736 can define a curved portion of the lumen 720 extending between the longitudinal portion of the lumen 720 and the side opening 712, thereby forming a curved path for the tether deployment assembly 760 as a portion of the tether deployment assembly 760 is advanced through the side opening 712. In some instances, the degree of curvature in the curvature of the curved wall portion 736 can be greater than that of the curvature in the curved wall portion 636 described with reference to the elongate shaft 602 of Figure 6. The degree of curvature of a curved wall portion 736 can be selected based at least in part on the patient anatomy. In some instances, the portion of the tether deployment assembly 760 disposed externally of the side opening 712 can be more proximally oriented than the portion of the tether deployment assembly 660 described with reference to Figure 6. For example, a distal portion 764, including a distal end 768, of the tether deployment assembly 760 disposed externally of the side opening 712 can be oriented more proximally than the distal end 668 of the tether deployment assembly 660 disposed externally of the side opening 612.

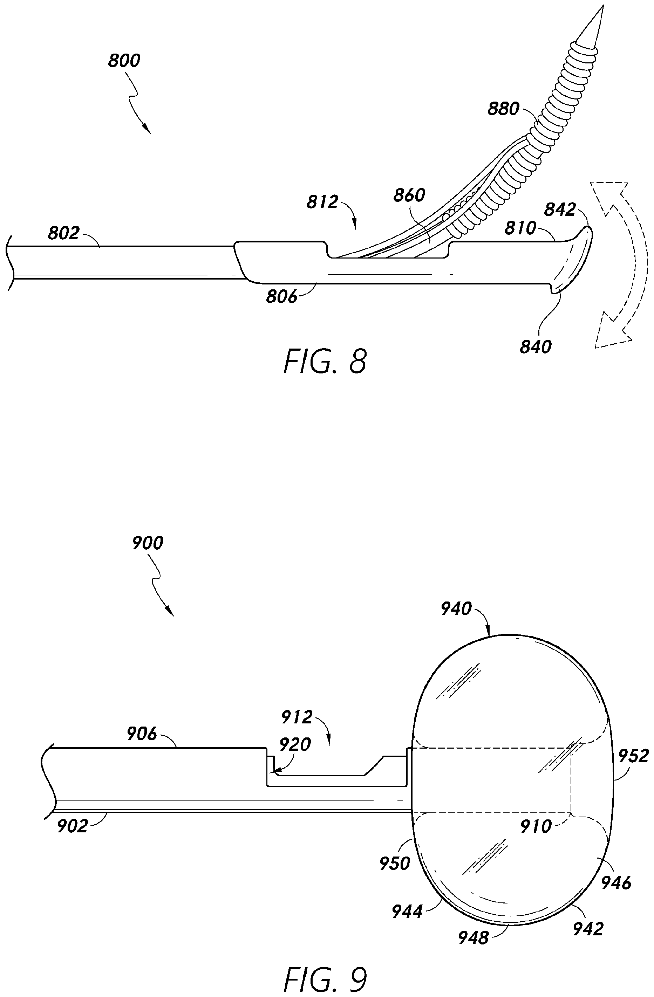

Figure 8 is a side perspective view of an example of an elongate shaft 802 of a tether deployment device 800. The tether deployment device 800 can comprise an atraumatic distal effector 840 coupled to a distal portion 806 of the elongate shaft 802. In some instances, at least a portion of the distal portion 806 of the elongate shaft 802 can be flexible, deformable, compressible and/or pliable such that an orientation of the atraumatic distal effector 840 relative to the elongate shaft 802 can be adjustable. For example, the orientation of the atraumatic distal effector 840 can change as contact is made with a first tissue area to facilitate desired positioning of the elongate shaft 802, including the side opening 812, relative to a target tissue area. Ability to change the orientation of an atraumatic distal effector 840 relative to the elongate shaft 802 can facilitate desired positioning of the atraumatic distal effector 840 against the first tissue area while the side opening 812 is oriented toward the target tissue area, thereby facilitating deployment of a tether from the elongate shaft 802. A portion of a tether deployment assembly 860 is shown as being disposed through the side opening 812 such that a portion of a tether 880 carried by the tether deployment assembly 860 can be deployed to the target tissue area.

In some instances, the atraumatic distal effector 840 can comprise a distally oriented surface 842, including a distally oriented planar or substantially planar surface configured to engage with a first tissue area adjacent to the target tissue area. In some instances, the atraumatic distal effector 840 can comprise disc comprising a distally oriented surface. In some instances, the atraumatic distal effector 840 can comprise a disc configuration. One or more portions of the atraumatic distal effector 840 can be flexible. For example, the atraumatic distal effector 840 can comprise a flexible disc coupled to the distal portion 806 of the elongate shaft 802, including a distal end 810 of the elongate shaft 802. In some instances, a portion of the elongate shaft 802 between the side opening 812 and the atraumatic distal effector 840, including a portion between the side opening 812 and the distal end 810 of the elongate shaft 802, can be flexible, deformable, compressible and/or pliable. In some instances, an orientation of the atraumatic distal effector 840 can rotate about one or both axes perpendicular or substantially perpendicular to the longitudinal axis of the elongate shaft 802. For example, the flexible disc can be rotatable about one or both axes perpendicular or substantially perpendicular to the longitudinal axis of the elongate shaft 802. The arrow in Figure 8 shows rotation of the atraumatic distal effector 840 in one plane relative to the elongate shaft 802. In some instances, while the atraumatic distal effector 840 is not in contact with a tissue area, the atraumatic distal effector 840 can have an orientation perpendicular or substantially perpendicular to the longitudinal axis of the elongate shaft 802. For example, while the portion of the distal portion 806 is in a relaxed state, such as not a compressed, deformed and/or bent configuration, the atraumatic distal effector 840 can have the perpendicular or substantially perpendicular orientation. While the portion of the distal portion 806 is in a compressed, deformed and/or bent configuration, the atraumatic distal effector 840 can be offset and/or rotated away from the perpendicular or substantially perpendicular orientation. The orientation of the atraumatic distal effector 840 can change upon contact with a first tissue area to provide desired placement of the elongate shaft 802 and/or side opening 812 relative to the target tissue area.

Alternatively, the atraumatic distal effector 840 can have an orientation other than being perpendicular or substantially perpendicular to the elongate shaft 802 while the atraumatic distal effector 840 is not in contact with a tissue area. The orientation of the atraumatic distal effector 840 may or may not be adjustable after contact with a first tissue area.

Figure 9 is a side perspective view of an example of a portion of a tether deployment device 900 that includes an atraumatic distal effector 940 comprising an inflatable balloon 942. A distal portion 906 of an elongate shaft 902 is shown. A side opening 912 is disposed on the distal portion 906 of the elongate shaft 902 and is in communication with a lumen 920 extending from a proximal end of the elongate shaft 902. A tether deployment assembly carrying a tether can be slidably received within the lumen 920, a portion of the tether deployment assembly and the tether can be deployed from the elongate shaft 902 through the side opening 912.

The inflatable balloon 942 can be coupled to the distal portion 906. For example, the inflatable balloon 942 can be positioned at least partially around the distal portion 906. In some instances, the inflatable balloon 942 can be positioned circumferentially around a portion of the elongate shaft 902 distal of the side opening 912. The inflatable balloon 942 can be circumferentially coupled to the distal portion 906 of the elongate shaft 902. The inflatable balloon 942 can be inflated prior to positioning a portion of the inflatable balloon 942 against a first tissue area. For example, the inflatable balloon 942 can be delivered to the target site in a deflated state. The inflatable balloon 942 can subsequently be inflated prior to contacting the inflatable balloon 942 to the first tissue area. Figure 9 shows the inflatable balloon 942 in an inflated state. In some instances, the inflatable balloon 942 can assume a ring shape in the inflated state. While the inflatable balloon 942 is in the inflated state, the inflatable balloon 942 can comprise a proximal portion 944, a distal portion 946 and a lateral portion 948 extending between the proximal portion 944 and the distal portion 946. In some instances, while the inflatable balloon 942 is in the inflated state, one or more of the lateral portion 948 and distal portion 946 can be in contact with a first tissue area adjacent to a target tissue area. In some instances, while the inflatable balloon 942 is in the inflated state, a distal end 952 of the inflatable balloon 942 can be distal of a distal end 910 of the elongate shaft 902. A proximal end 950 of the inflatable balloon 942 can be distal of the side opening 912. The distal end 952 of the inflatable balloon 942 can be configured to contact the first tissue area tissue adjacent to the target tissue area. In some instances, the distal end 952 being distal of the distal end 910 of the elongate shaft 902 can prevent or reduce injury to the tissue.

In some instances, the tether deployment device 700, 800, 900 can comprise one or more other features of the tether deployment device 600 described with reference to Figure 6. For example, the elongate shafts 702, 802, 902 can comprise one or more other features of the elongate shaft 602. In some instances, each of the elongate shafts 702, 802, 902 can comprise all the other features of the elongate shaft 602. For example, elongate shaft 702 can have the same features as those of elongate shaft 602, aside from the degree of curvature of the curved wall portion 736. Each of the elongate shafts 802, 902 can be the same as or similar to the elongate shaft 602, aside from features of the distal effectors 840, 940. In some instances, each of the elongate shafts 702, 802, 902 can extend distally from a respective handle having the same or similar features as the handle 500 describe with reference to Figure 5.

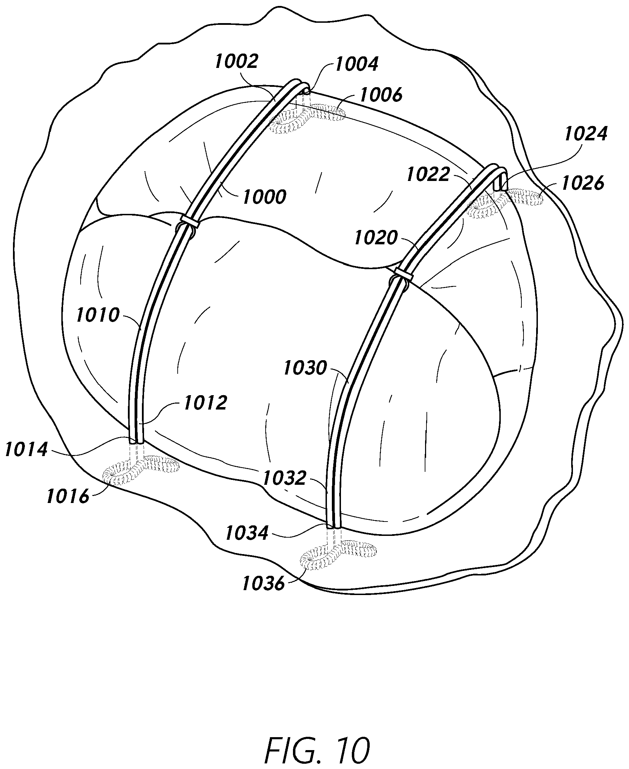

As described herein, a process for repairing a heart valve can comprise tethering an anterior portion and a posterior portion of a heart valve annulus to one another. For example, distal portions of one or more tethers can be deployed to respective positions on the anterior portion and distal portions of one or more tethers can be deployed to respective positions on the posterior portion. A proximal portion of a tether deployed to the anterior portion can be coupled to a proximal portion of a respective tether deployed to the posterior portion. Figure 10 is a top-down perspective view of a plurality of tethers coupling an anterior portion of a mitral valve annulus to a posterior portions of the annulus. An atrium-oriented view is shown. Two sets of tethers can couple respective anterior and posterior portions of the annulus toward one another. A distal portion 1004 of a first tether 1000 can be positioned over a surface of a first portion of the annulus oriented toward a ventricle, such as a left ventricle. A distal portion 1014 of a second tether 1010 can be positioned over a surface of a second portion of the annulus oriented toward the ventricle. For example, the first portion of the annulus can be a posterior portion of the annulus. The second portion of the annulus can be an anterior portion of the annulus. The proximal portion 1002 of the first tether 1000 and the proximal portion 1012 of the second tether 1010 can be coupled together over an atrium-facing portion of the heart valve. The first and second tethers 1000, 1010 can extend from the ventricle-facing surface through the annulus to the atrium-facing surface. Coupling the proximal portion 1002 of the first tether 1000 and the proximal portion 1012 of the second tether 1010 can pull the anterior portion and the posterior portion of the annulus toward one another. In some instances, the second portion of the annulus can be opposite that of the first portion of the annulus such that the first tether 1000 and the second tether 1010 can pull opposing portions of the annulus toward one another.

A distal portion 1024 of a third tether 1020 can be positioned over a surface of a third portion of the annulus oriented toward the ventricle. A distal portion 1034 of a fourth tether 1030 can be positioned over a surface of a fourth portion of the annulus oriented toward the ventricle. The third portion of the annulus can be a posterior portion of the annulus. The fourth portion of the annulus can be an anterior portion of the annulus. The proximal portion 1022 of the third tether 1020 and the proximal portion 1032 of the fourth tether 1030 can be coupled together over an atrium-facing portion of the heart valve. The third and fourth tethers 1020, 1030 can each extend from the ventricle-facing surface through the annulus to the atrium-facing surface. In some instances, the third portion and fourth portion of the annulus can be opposing portions of the annulus, for example, the third and fourth portions of the annulus can be opposing portions of the posterior and anterior portions of the annulus.

Distal portions 1004, 1014, 1024, 1034 of the first, second, third and fourth tethers 1000, 1010, 1020, 1030 can each comprise an anchor 1006, 1016, 1026, 1036, such as a knot formed using a portion of the respective tether 1000, 1010, 1020, 1030.

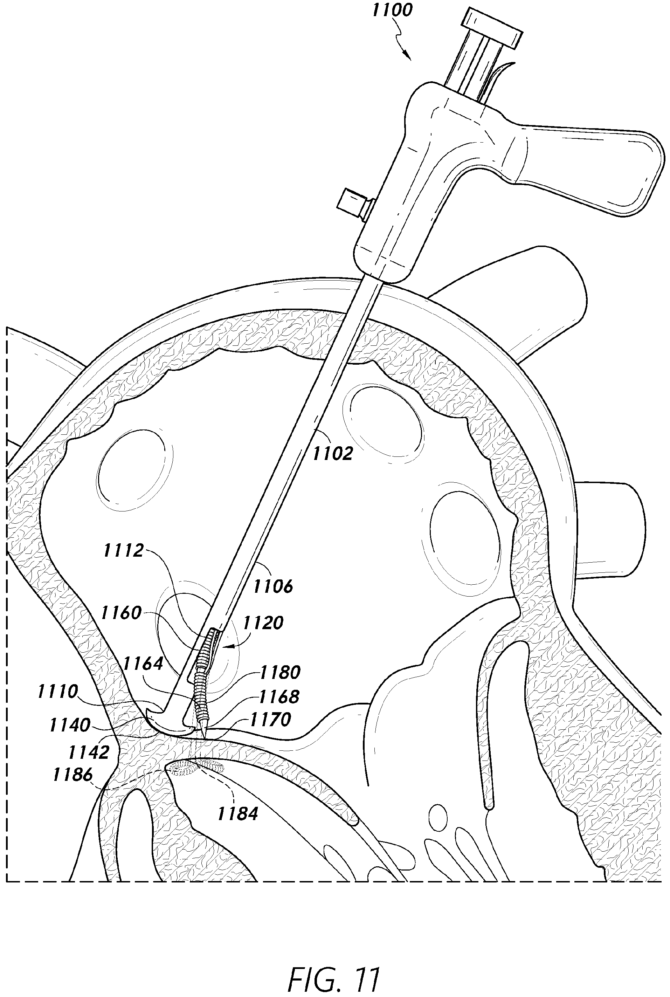

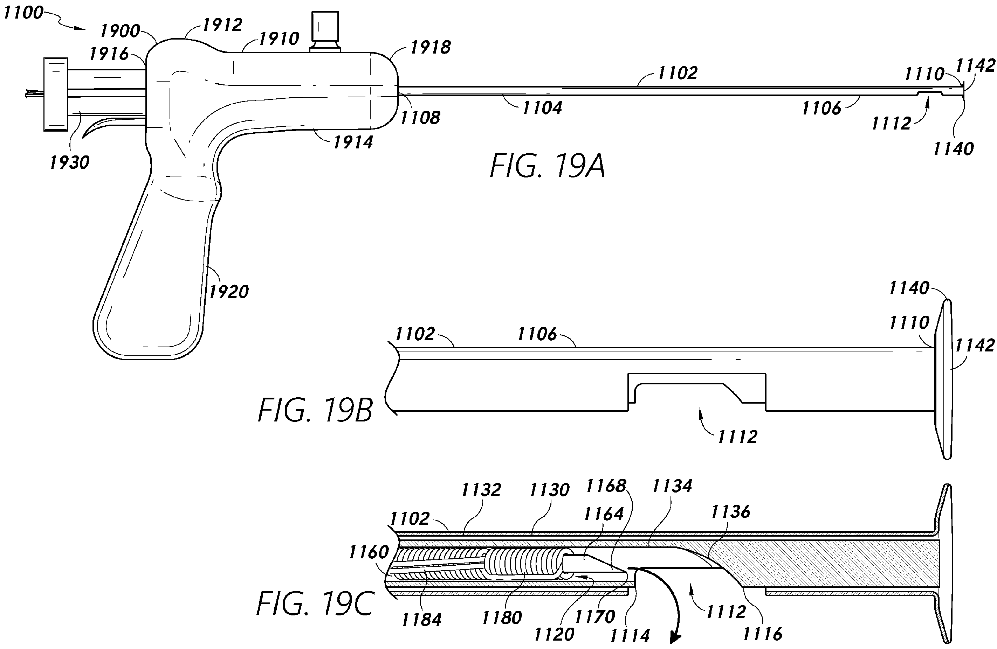

Figure 11 shows deployment of a tether to an annulus of a heart valve, such as a mitral valve, using a tether deployment device 1100 by accessing the annulus through an atrium, such as the left atrium. As described in further detail herein, the tether deployment device 1100 can comprise an elongate shaft 1102 comprising a lumen 1120 extending from a proximal end 1108 (as shown in Figure 19) to a side opening 1112 on a distal portion 1106 thereof. An atraumatic distal effector 1140 can be associated with the distal portion 1106 of the elongate shaft 1102 distally of the side opening 1112, for example being coupled to a distal end 1110 of the elongate shaft 1102. A tether deployment assembly 1160 can be configured to be at least partially received within the lumen 1120. The tether deployment assembly 1160 can have a tether 1180 comprising a distal portion 1184 configured to be deployed through the side opening 1112 to a target tissue area of the annulus.

At least a portion of the elongate shaft 1102 can be advanced into the left atrium, such as through an opening formed on a left atrial wall. For example, a distal portion 1106 of the elongate shaft 1102 can be inserted through the opening formed in the left atrial wall and advanced toward the target position on the annulus. The elongate shaft 1102 can be advanced into the left atrium until the atraumatic distal effector 1140 contacts a first tissue area adjacent to a target tissue area on the annulus. In some instances, the atraumatic distal effector 1140 can contact tissue adjacent to the annulus and/or a portion of the annulus. Advancement of the elongate shaft 1102 into the left atrium and/or placement of the atraumatic distal effector 1140 against the first tissue area can be guided using any number of imaging techniques, including for example transesophageal echocardiography (TEE), intracardiac echocardiography (ICE) and/or fluoroscopy. The side opening 1112 can be configured to be oriented toward a target portion of the mitral valve annulus facing the atrium, while the atraumatic distal effector 1140 is in contact the first tissue area. In some instances, the first tissue area can comprise an atrial wall tissue and/or an area of heart tissue between the atrial wall and the annulus. The target tissue area can comprise an atrial-facing portion of the annulus. For example, the target tissue area can be on a posterior portion of the annulus or an anterior portion of the annulus. In some instances, the first tissue area can comprise heart tissue on an area of heart tissue between the atrial wall and a position on the posterior portion or anterior portion of the annulus, such as tissue at a junction of the atrial wall and the position on the posterior portion or anterior portion of the annulus.

While a distally oriented surface 1142 of the atraumatic distal effector 1140 is in contact with the first tissue area and the side opening 1112 of elongate shaft 1102 is oriented toward the target tissue, a portion of the tether deployment assembly 1160, including a distal portion 1164, can be advanced through the side opening 1112 of the elongate shaft 1102. An exit trajectory of the tether deployment assembly 1160 through the side opening 1112 can be at an angle relative to the elongate shaft 1102. In some instances, the tether deployment assembly 1160 can comprise a needle 1170 at a distal end 1168. The distal portion 1164 can be advanced out of the side opening 1112 such that the needle 1170 can puncture and be advanced through the target tissue area from a first surface, such as an atrial-facing surface, to a second surface, such as a ventricle-facing surface, of the annulus. The target tissue area can be adjacent to the first tissue area contacted by the atraumatic distal effector 1140. The exit trajectory of the tether deployment assembly 1160 can be away from a heart wall portion, including an atrial and/or ventricular wall portion, to avoid accidental puncture of the heart wall. A portion of the tether 1180, including the distal portion 1184 of the tether 1180, can be deployed from the tether deployment assembly 1160 to form an anchor 1186 over the ventricle-facing surface of the annulus at the target location.

The anchor 1186 can have one or more features of other anchors described herein. For example, the anchor 1186 can be formed using the distal portion 1184 of the tether 1180. The anchor 1186 can comprise a knot formed using the distal portion 1184 of the tether 1180. The anchor 1186 can be integral with the remainder of the tether 1180. In some instances, the tether 1180 can comprise a suture. The anchor 1186 can comprise a suture knot, including a bulky suture knot. In some instances, the suture knot, such as the bulky suture knot, can be formed using a distal portion of the suture and be integral with the remainder of the suture. The tether 1180 can comprise for example expanded polytetrafluoroethylene (ePTFE). The tethers 1180 can be an ePTFE suture. In some instances, the tether 1180 can comprise a polybutilate-coated polyester suture. In some instances, the tether 1180 can comprise malleable stainless steel, a shape memory material and/or a superelastic alloy.

The tether deployment assembly 1160 can be retracted after the anchor 1186 is deployed. For example, the tether deployment assembly 1160 can be withdrawn back into the lumen 1120 of the elongate shaft 1102. The elongate shaft 1102 can be withdrawn back through the opening formed in the atrial wall. As the elongate shaft 1102 is retracted, additional portions of the tether 1180 can be extended out through the side opening 1112. A proximal portion 1182 (not shown) of the tether 1180 can remain within the left atrium for coupling to a tether deployed to another portion of the annulus. For example, the anchor 1186 can be formed over an anterior portion of the annulus. The proximal portion 1182 of the tether 1180 can be coupled to a proximal portion of a tether comprising a distal portion deployed to a posterior portion of the annulus.

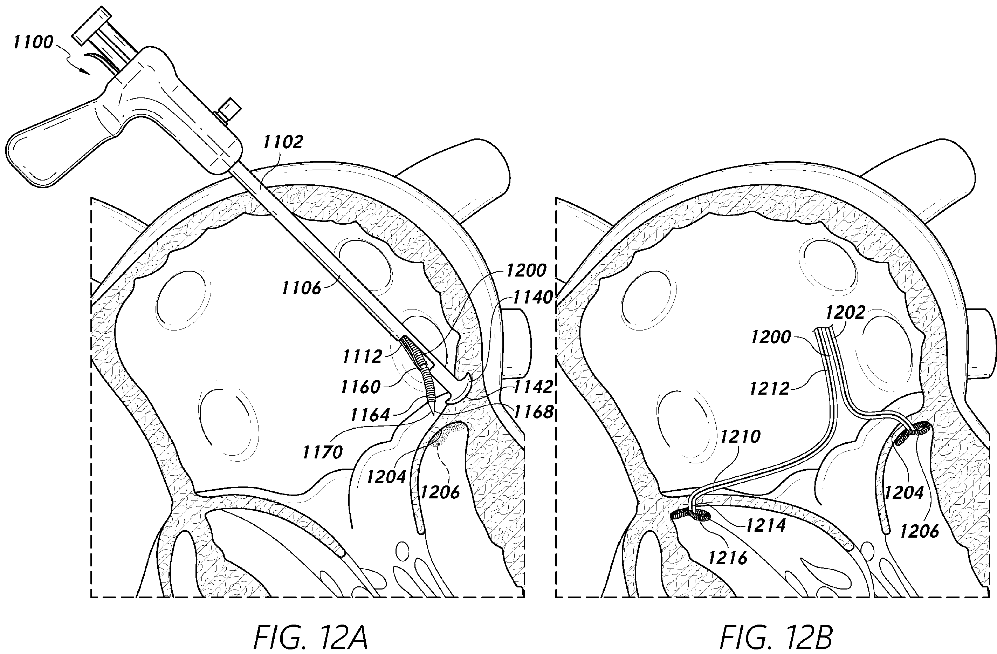

Figures 12A and 12B show a process of deploying a first tether 1200 and a second tether 1210 to a first position and a second position on an annulus, respectively. In some instances, the first position can be on a posterior portion of the annulus, and the second position can be on an anterior portion of the annulus, or vice versa. In some instances, the first and second positions can be at opposing or substantially opposing portions of the annulus. The two tethers 1200, 1210 can be coupled to one another to pull the posterior and anterior portions, including opposing posterior and anterior portions, of the annulus toward one another.

The deployment process can comprise one or more steps the same as or similar to those described with reference to Figure 11. For example, the tether deployment device 1100 can be used for the deployment of the first and second tethers 1200, 1210. Referring to Figure 12A, the first tether 1200 can be deployed to the first position. A portion of the elongate shaft 1102 of the tether deployment device 1100 can be advanced into the left atrium. The distally oriented surface 1142 of the atraumatic distal effector 1140 can be positioned against a first tissue area. For example, the first tissue area can comprise a junction between the atrial wall and the first position on the annulus. In some instances, the atraumatic distal effector 1140 can contact tissue adjacent to the annulus and/or a portion of the annulus. A portion of the tether deployment assembly 1160, such as a distal portion 1164, can be advanced through the side opening 1112 of the elongate shaft 1102 such that the needle 1170 at the distal end 1168 of the tether deployment assembly 1160 can puncture and be advanced through the target tissue area from a first surface, such as an atrial-facing surface, to a second surface, such as a ventricle-facing surface, of the annulus. The target tissue area can be adjacent to the first tissue area contacted by the atraumatic distal effector 1140. A portion of the tether 1200, including the distal portion 1204 of the tether 1200, can be deployed from the tether deployment assembly 1160 to form an anchor 1206 over the ventricle-facing surface of the annulus at the first position. As described herein, the anchor 1206 can be formed using the distal portion 1204 of the tether 1200.

The tether deployment assembly 1160 can be retracted back into the elongate shaft 1102 and the elongate shaft 1102 can be retracted back through the opening formed in the atrial wall, after the anchor 1206 is deployed. As the elongate shaft 1102 is retracted, additional portions of the tether 1200 can be extended out through the side opening 1112. A proximal portion 1202 of the tether 1200 can remain within the left atrium for coupling to the second tether 1210.

Referring to Figure 12B, the second tether 1210 can be deployed to the second position. Deployment of the second tether 1210 can be similar to or the same as that for the first tether 1200. A distal portion 1214 of the tether 1210 can be used to form an anchor 1216 over the ventricle-facing surface of the annulus at second position. A proximal portion 1212 of the second tether 1210 can remain in the atrium such that it can be coupled the proximal portion 1202 of the first tether 1200. In Figure 12B, a portion of the first tether 1200 can be extended toward the second tether 1210 such that the first and second tethers 1200, 1210 can be coupled to one another over a posterior portion of the annulus. For example, the first and second tethers 1200, 1210 can be coupled to one another over the first position on the posterior portion of the annulus. In some instances, the first tether 1200 can be tensioned to pull the anterior portion of the annulus toward the posterior portion of the annulus.

The first and second tethers 1200, 1210 can be coupled together using any number of techniques and/or fasteners. In some instances, the first and second tethers 1200, 1210 can be knotted together. In some instances, the first and second tethers 1200, 1210 can be coupled together using one or more mechanical fasteners, including a clip, tie and/or clasp. In some instances, the first and second tethers 1200, 1210 can be coupled together using one or more methods and/or devices described with in U.S. Patent No. 11,065,120, entitled "Method and Apparatus for Cardiac Procedures", and issued on July 20, 2021.

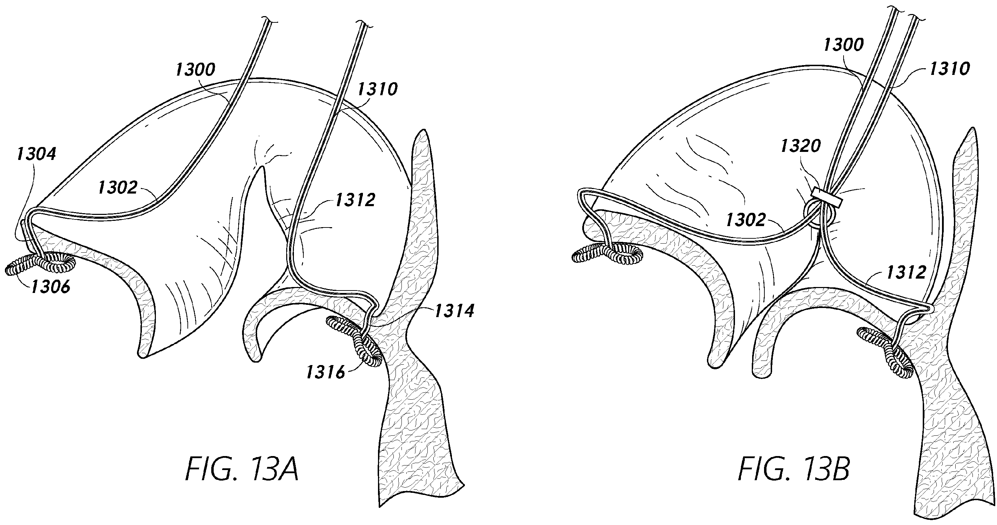

Figures 13 to 18 show various examples of tethers deployed to both posterior and anterior portions of the mitral valve annulus. An atrium-facing view of the heart valve is shown in each of the figures. It will be understood that the figures are for illustrative purposes only. More or fewer tethers can be deployed. Tethers can be deployed to other positions on the mitral valve annulus.

Figures 13A and 13B show an example of deployment of two tethers to an anterior portion and a posterior portion of an annulus of a mitral valve. Figure 13A shows a distal portion 1304 of a first tether 1300 deployed to an anterior portion of the annulus. The distal portion 1304 of the first tether 1300 can be positioned over a surface of an anterior portion of the annulus oriented toward a left ventricle. A distal portion 1314 of a second tether 1310 can be deployed to a posterior portion of the annulus. The distal portion 1314 of the second tether 1310 can be positioned over a surface of a posterior portion of the annulus oriented toward the ventricle. Respective anchors 1306, 1316 can be associated with the distal portions 1304, 1314. For example, distal portions 1304, 1314 can comprise the respective anchor 1306, 1316. The anchors 1306, 1316 can each comprise a knot formed using a portion of the respective tethers 1300, 1310. For example, the knot can be a bulky suture knot. In some instances, the distal portion 1304 of the first tether 1300 can be coupled to a portion of the annulus adjacent to an anteromiddle portion of the anterior mitral valve leaflet. In some instances, the distal portion 1314 of the second tether 1310 can be coupled to a portion of the annulus adjacent to a posteromiddle scallop portion of the posterior mitral valve leaflet. In some instances, the distal portion 1304 of the first tether 1300 and the distal portion 1314 of the second tether 1310 can be at opposing or substantially positions on the annulus.

Figure 13B shows that after deployment of the distal portion 1304 of the first tether 1300 and the distal portion 1314 of the second tether 1310 to their respective locations on the annulus, the proximal portion 1302 of the first tether 1300 and the proximal portion 1312 of the second tether 1310 can be coupled together over an atrium-facing portion of the heart valve. The first and second tethers 1300, 1310 can extend from the ventricle-facing surface through the annulus to the atrium-facing surface. In some instances, the proximal portion 1302 of the first tether 1300 and the proximal portion 1312 of the second tether 1310 can be coupled together over a coaptation zone of the mitral valve leaflets. In some instances, the proximal portions 1302, 1312 can be tensioned and coupled together over the coaptation zone to pull the respective portions of the anterior and posterior annulus portions toward one another. A fastening component 1320 can be used to couple the two tethers 1300, 1310 together. The fastening component 1320 can be formed using any number of techniques and/or comprise any number of fasteners.

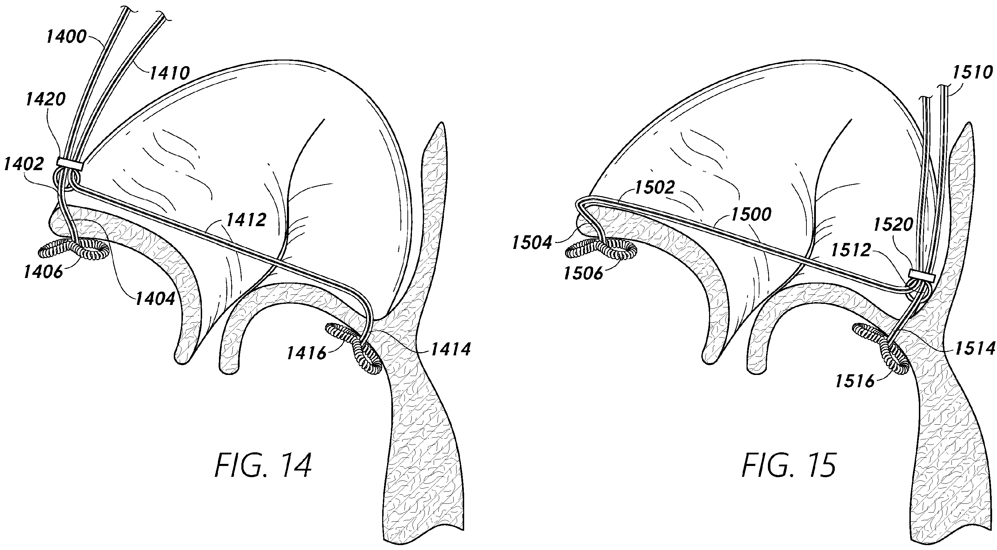

Figures 14 and 15 show examples of deploying a first tether 1400, 1500 to an anterior portion and a second tether 1410, 1510 to a posterior portion of an annulus of a mitral valve, where the first tether 1400, 1500 and the second tether 1410, 1510 are coupled to one another over an anterior portion of the annulus or over a posterior portion of the annulus, respectively. Each tether can be deployed to a respective position of the annulus such that a distal portion 1404, 1504 of the first tether 1400, 1500 can be positioned over a surface of an anterior portion of the annulus oriented toward a left ventricle. The distal portion 1414, 1514 of the second tether 1410, 1510 can be positioned over a surface of a posterior portion of the annulus oriented toward a left ventricle. The distal portions 1404, 1504, 1414, 1514 can each comprise a respective anchor 1406, 1416, 1506, 1516 positioned over a surface of a posterior portion of the annulus oriented toward a left ventricle. As described herein, the anchors 1406, 1416, 1506, 1516 can each comprise a knot formed using a portion of the respective tethers. For example, the knot can be a bulky suture knot. The first tethers 1400, 1500 and second tethers 1410, 1510 can extend from the ventricle-facing surface through the annulus to the atrium-facing surface. The proximal portion 1402, 1502 of the first tether 1400, 1500 and the proximal portion 1412, 1512 of the second tether 1410, 1510 can be coupled together over an atrium-facing portion of the heart valve. Respective fastening components 1420, 1520 can be used to couple the corresponding tethers together, including fastening components formed using any number of techniques and/or fastening components comprising any number of fasteners.

In some instances, the distal portion 1404, 1504 of the first tether 1400, 1500 can be coupled to a portion of the annulus adjacent to an anteromiddle portion of the anterior mitral valve leaflet. In some instances, the distal portion 1414, 1514 of the second tether 1410, 1510 can be coupled to a portion of the annulus adjacent to a posteromiddle scallop portion of the posterior mitral valve leaflet. In some instances, the distal portion 1404, 1504 of the first tether 1400, 1500 and the distal portion 1414, 1514 of the second tether 1410, 1510 can be at opposing or substantially positions on the annulus.

Referring to Figure 14, the proximal portion 1402 of the first tether 1400 and the proximal portion 1412 of the second tether 1410 can be coupled together over the location of the annulus to which the distal portion 1404 of the first tether 1400 is coupled. In some instances, the first tether 1400 and the second tether 1410 can be coupled together over a portion of the annulus adjacent to the anteromiddle portion of the anterior mitral valve leaflet. In some instances, the proximal portion 1412 of the second tether 1410 can be tensioned. The proximal portion 1412 of the second tether 1410 can be pulled toward the first tether 1400 anchored to the anteromiddle portion of the anterior mitral valve leaflet and coupled to the first tether 1400 over the anteromiddle portion of the anterior mitral valve leaflet so as to pull the posteromiddle scallop of the posterior mitral valve leaflet toward the anteromiddle portion of the anterior mitral valve leaflet.

Referring to Figure 15, the proximal portion 1502 of the first tether 1500 and the proximal portion 1512 of the second tether 1510 can be coupled together over the location of the annulus to which the distal portion 1514 of the second tether 1510 is coupled. In some instances, the first tether 1500 and the second tether 1510 can be coupled together over a portion of the annulus adjacent to the posteromiddle scallop of the posterior mitral valve leaflet. The proximal portion 1502 of the first tether 1500 can be pulled toward the second tether 1510 anchored to the posteromiddle scallop of the posterior mitral valve leaflet and coupled to the second tether 1510 over the posteromiddle scallop of the posterior mitral valve leaflet so as to pull the anteromiddle portion of the mitral valve leaflet toward the posteromiddle scallop of the posterior mitral valve leaflet.

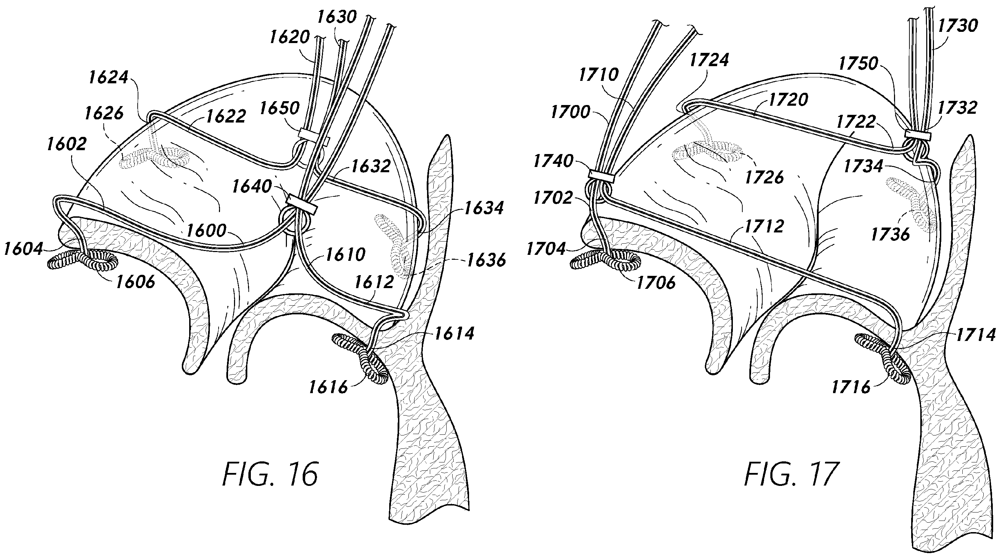

Figures 16, 17 and 18 each show examples of four tethers deployed to a mitral valve annulus to pull respective anterior and posterior portions of the annulus toward one another. In each of the figures, a distal portion 1604, 1704, 1804 of a first tether 1600, 1700, 1800 can be deployed to a first position on an anterior portion of the annulus and a distal portion 1614, 1714, 1814 of a second tether 1610, 1710, 1810 can be deployed to a first position on a posterior portion of the annulus. A distal portion 1624, 1724, 1824 of a third tether 1620, 1720, 1820 can be deployed to a second position on the anterior portion of the annulus and a distal portion 1634, 1734, 1834 of a fourth tether 1630, 1730, 1830 can be deployed to a second position on the posterior portion of the annulus. Respective anchors 1606, 1616, 1626, 1636, 1706, 1716, 1726, 1736, 1806, 1816, 1826, 1836 can be associated with the distal portions 1604, 1614, 1624, 1634, 1704, 1714, 1724, 1734, 1804, 1814, 1824, 1834. For example, distal portions 1604, 1614, 1624, 1634, 1704, 1714, 1724, 1734, 1804, 1814, 1824, 1834 can comprise the respective anchor 1606, 1616, 1626, 1636, 1706, 1716, 1726, 1736, 1806, 1816, 1826, 1836. The anchors 1606, 1616, 1626, 1636, 1706, 1716, 1726, 1736, 1806, 1816, 1826, 1836 can each comprise a knot formed using a portion of the respective tethers 1600, 1610, 1620, 1630, 1700, 1710, 1720, 1730, 1800, 1810, 1820, 1830. For example, the knot can be a bulky suture knot. The first position and second position on the anterior portion of the annulus can be spaced from one another. The first position and second position on the posterior portion of the annulus can be spaced from one another. Spacing of the positions on the anterior and posterior portions of the annulus can be selected based on anatomical needs of the patient.

In some instances, the first position on the anterior portion of the annulus can be a portion of the annulus adjacent to an anteromiddle portion of the anterior mitral valve leaflet. In some instances, the first position on the posterior portion of the annulus can be a portion of the annulus adjacent to a posteromiddle scallop portion of the posterior mitral valve leaflet. In some instances, the first positions can be at opposing positions on the annulus. In some instances, the second position on the anterior portion of the annulus can be a portion of the annulus adjacent to an anteromedial portion of the anterior mitral valve leaflet. In some instances, the second position on the posterior portion of the annulus can be a portion of the annulus adjacent to a posteromedial scallop portion of the posterior mitral valve leaflet. In some instances, the second positions can be at opposing positions on the annulus.

Each of the first tethers 1600, 1700, 1800, second tethers 1610, 1710, 1810, third tethers 1620, 1720, 1820 and fourth tethers 1630, 1730, 1830 can extend from the ventricle-facing surface through the annulus to the atrium-facing surface. Respective portions of the proximal portions 1602, 1702, 1802 of the first tethers 1600, 1700, 1800 and proximal portions 1612, 1712, 1812 of the second tethers 1610, 1710, 1810 can be coupled together, over an atrium-facing portion of the heart valve. Respective portions of the proximal portions 1622, 1722, 1822 of the third tethers 1620, 1720, 1820 and the proximal portions 1632, 1732, 1832 of the fourth tethers 1630, 1730, 1830 can be coupled together, over an atrium-facing portion of the heart valve.

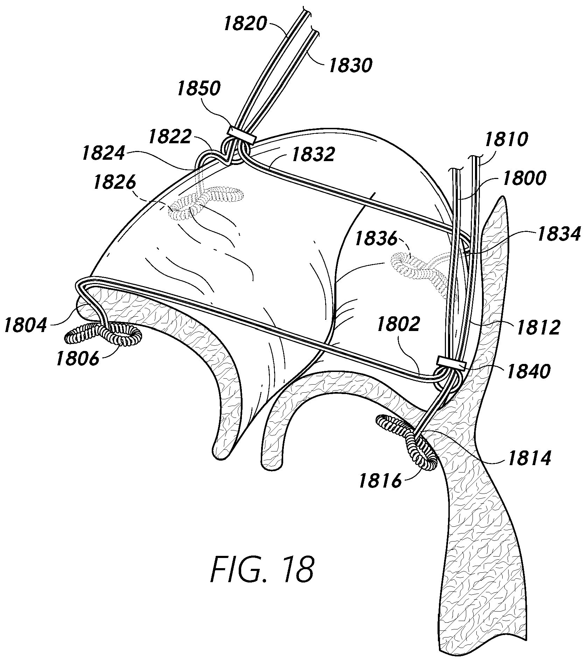

Referring to Figure 16, the first and second tethers 1600, 1610, and the third and fourth tethers 1620, 1630, can be coupled to one another over a coaptation zone of the heart valve leaflets. Referring to Figure 17, the first and second tethers 1700, 1710 can be coupled to one another over an anterior portion of the annulus. In some instances, the first and second tethers 1700, 1710 can be coupled to one another over the first position on the anterior portion of the annulus. The third and fourth tethers 1720, 1730 can be coupled to one another over a posterior portion of the annulus. In some instances, the third and fourth tethers 1720, 1730 can be coupled to one another over the second position on the posterior portion of the annulus. In Figure 18, the first and second tethers 1800, 1810 can be coupled to one another over a posterior portion of the annulus. In some instances, the first and second tethers 1800, 1810 can be coupled to one another over the first position on the posterior portion of the annulus. The third and fourth tethers 1820, 1830 can be coupled to one another over an anterior portion of the annulus. In some instances, the third and fourth tethers 1820, 1830 can be coupled to one another over the second position on the anterior portion of the annulus.

Respective fastening components 1640, 1650, 1740, 1750, 1840, 1850 can be used to couple the corresponding tethers together, including fastening components formed using any number of techniques and/or fastening components comprising any number of fasteners. One or more positions of the annulus to which a tether is deployed can be chosen based at least on the anatomy of the heart valve, including a location of enlargement of the heart valve orifice. For example, portions of the annulus adjacent to the posteromiddle scallop of the posterior mitral valve leaflet and anteromiddle portion of the anterior mitral valve leaflet can be coupled together if the posterior and anterior leaflets do not coapt at a lateral region of the mitral valve. In some instances, portions of the annulus adjacent to the posteromedial scallop of the posterior mitral valve leaflet and anteromedial portion of the anterior mitral valve leaflet can be coupled together if the posterior and anterior leaflets do not coapt medially.

Figures 19A, 19B and 19C show various views of the tether deployment device 1100 described with reference to the deployment process of Figures 11 and 12. Figure 19A is a side view of the tether deployment device 1100. The tether deployment device 1100 can comprise a handle 1900 and the elongate shaft 1102 can extend distally from the handle 1900. A proximal portion 1104 of the elongate shaft 1102 can be coupled to the handle 1900. For example, the proximal end 1108 of the elongate shaft 1102 can be coupled to the handle 1900. The handle 1900 can comprise a longitudinal portion 1910 comprising a longitudinal axis that is parallel or substantially parallel to the longitudinal of the elongate shaft 1102. The longitudinal portion 1910 can extend along its longitudinal axis parallel or substantially parallel to the longitudinal axis of the elongate shaft 1102. The proximal end 1108 of the elongate shaft 1102 can extend from a distal portion 1914, including a distal end 1918, of the longitudinal portion 1910.

The handle 1900 can comprise a lateral portion 1920 extending laterally from the longitudinal portion 1910. The lateral portion 1920 can extend at an angle from the longitudinal portion 1910, for example extending at an angle relative to the longitudinal axis of the elongate shaft 1102. The lateral portion 1920 can be configured to engage with an operator. For example, an operator can grasp onto the lateral portion 1920 while manipulating the tether deployment device 1100. In some instances, the lateral portion 1920 can extend from a proximal portion 1912 of the longitudinal portion 1910.

The side opening 1112 of the elongate shaft 1102 can have an orientation the same as or similar to that of the lateral portion 1920. For example, the lateral portion 1920 can extend along a first direction relative to the longitudinal axis of the elongate shaft 1102 and the side opening 1112 can be on a portion of the elongate shaft 1102 oriented toward the same direction relative to the longitudinal axis of the elongate shaft 1102. The side opening 1112 can be oriented toward the same direction along which the lateral portion 1920 extends. For example, the side opening 1112 can be ventrally oriented on the elongate shaft 1102.

A plunger 1930 can be partially movably disposed within the handle 1900, for example comprising at least a portion extending proximally from a proximal end 1916 of the longitudinal portion 1910 of the handle 1900. The tether deployment assembly 1160 can be coupled to the plunger 1930. The plunger 1930 can be used to actuate or move the tether deployment assembly 1160 during deployment of the tether 1180. Alternatively or in combination, other means of actuating or moving the tether deployment assembly 1160 can be used, including a spring component, gear and rack, six bar linkage and/or electronic motor. In some instances, the spring component, gear and rack, six bar linkage and/or electronic motor can be at least partially housed within the handle 1900. In some instances, the tether deployment device 1100 can comprise a trigger mechanism, for example at least partially housed within the handle 1900, configured to actuate or move the tether deployment assembly 1160.

The tether deployment device 1100 can comprise one or more other features of the tether deployment device 400 described with reference to Figure 5. Referring to Figure 19B, a more detailed side view of the distal portion 1106 of the elongate shaft 1102 is shown. The ventrally oriented side opening 1112 is shown. The atraumatic distal effector 1140 can be coupled to at least a portion of the distal portion 1106 of the elongate shaft 1102 at a position that is distal of the side opening 1112. In some instances, the atraumatic distal effector 1140 can be at a distal end 1110 of the elongate shaft 1102. In some instances, the atraumatic distal effector 1140 can comprise a distally oriented planar or substantially planar surface 1142 configured to engage with a first tissue area adjacent to the target tissue area. The atraumatic distal effector 1140 can comprise one or more distally oriented round planar or substantially planar surfaces, such as one or more circular or substantially circular planar surfaces. In some instances, the atraumatic distal effector 1140 can comprise a disc configuration. The atraumatic distal effector 1140 can comprise a flexible disc coupled to the distal end 1110 of the elongate shaft 1102. A distally oriented surface of the flexible disc can be configured to contact the first tissue area. In some instances, the distally oriented surface of the atraumatic distal effector 1140 can be perpendicularly or substantially perpendicularly oriented relative to the longitudinal axis of the elongate shaft 1102. For example, the distally oriented surface of the disc, such as flexible disc, can be perpendicular or substantially perpendicular to the longitudinal axis.

Figure 19C is a side cross-section view of the distal portion 1106 of the elongate shaft 1102. The lumen 1120 of the elongate shaft 1102 can extend within the elongate shaft 1102 from the proximal end 1108 to the side opening 1112 on the distal portion 1106 of the elongate shaft 1102. An inner wall 1130 of the elongate shaft 1102 can define the lumen 1120. A longitudinal wall portion 1132 of the inner wall 1130 can define a longitudinal portion of the lumen 1120, for example extending from the proximal end 1108 of the elongate shaft 1102 to the proximal end 1114 of the side opening 1112. The longitudinal portion of the lumen 1120 can extend along an axis parallel or substantially parallel to a longitudinal axis of the elongate shaft 1102. The inner wall 1130 can have a curved wall portion 1136 proximate and/or adjacent to the side opening 1112. For example, a distal portion 1134 of the inner wall 1130 can comprise the curved wall portion 1136. The curved wall portion 1136 can be distal of the longitudinal wall portion 1132 and define a curved path extending between the longitudinal portion of the lumen 1120 and the side opening 1112. In some instances, the curved wall portion 1136 can extend from the longitudinal wall portion 1132 to the side opening 1112. In some instances, the curved wall portion 1136 can be on a portion of the inner wall 1130 opposite that of the side opening 1112. For example, the curved wall portion 1136 can extend from a portion of the inner wall 1130 opposite the proximal end 1114 of the side opening 1112 to a portion of the inner wall 1130 opposite a distal end 1116 of the side opening 1112.

The tether deployment assembly 1160 comprising the tether 1180, can be configured to be at least partially received within the lumen 1120. The tether deployment assembly 1160 can comprise a needle 1170 at a distal end 1168. The arrow shows advancement of a portion the tether deployment assembly 1160 through the side opening 1112. One or more portions of the tether deployment assembly 1160, including the distal portion 1164 of the tether deployment assembly 1160, can glide and/or slide over and/or along the curved wall portion 1136 as the distal portion 1164 is advanced to the distal end of the lumen 1120 and out of the side opening 1112. The curved wall portion 1136 can be configured to guide the exit trajectory of the distal portion 1164 of the tether deployment assembly 1160 through the side opening 1112.

In some instances, the tether deployment device 1100 can comprise one or more other features of tether deployment devices 600, 700, 800, 900 described with reference to Figures 6, 7, 8 and 9. For example, the distal portion 1134 of the inner wall 1130 can comprise a groove configured to guide movement of the portion of the tether deployment assembly 1160 through the lumen 1120 as the portion of the tether deployment assembly 1160 is advanced distally to the side opening 1112. The groove can be on at least a portion of the curved wall portion 1136, including on a portion of the inner wall 1130 opposite the side opening 1112. For example, at least a portion of the curved wall portion 1136 can define the groove. The groove can have the same or similar orientation as the curvature of the curved wall portion 1136. For example, the groove can extend along a direction parallel or substantially parallel to the curvature of the curved wall portion 1136 extending from the longitudinal wall portion 1132 to the side opening 1112. The portion of the tether deployment assembly 1160, such as at least a portion of the distal portion 1164 of the tether deployment assembly 1160 can glide and/or slide over and/or along at least a portion of the groove as the portion is advanced to the side opening 1112.

In some instances, at least a portion of the distal portion 1106 of the elongate shaft 1102, including a portion between the side opening 1112 and the distal end 1110 of the elongate shaft 1102, can be flexible, deformable, compressible and/or pliable such that an orientation of the atraumatic distal effector 1140 relative to the elongate shaft 1102 can be adjustable. In alternative examples, the atraumatic distal effector 1140 can comprise an inflatable balloon having one or more features as described with reference to Figure 9.

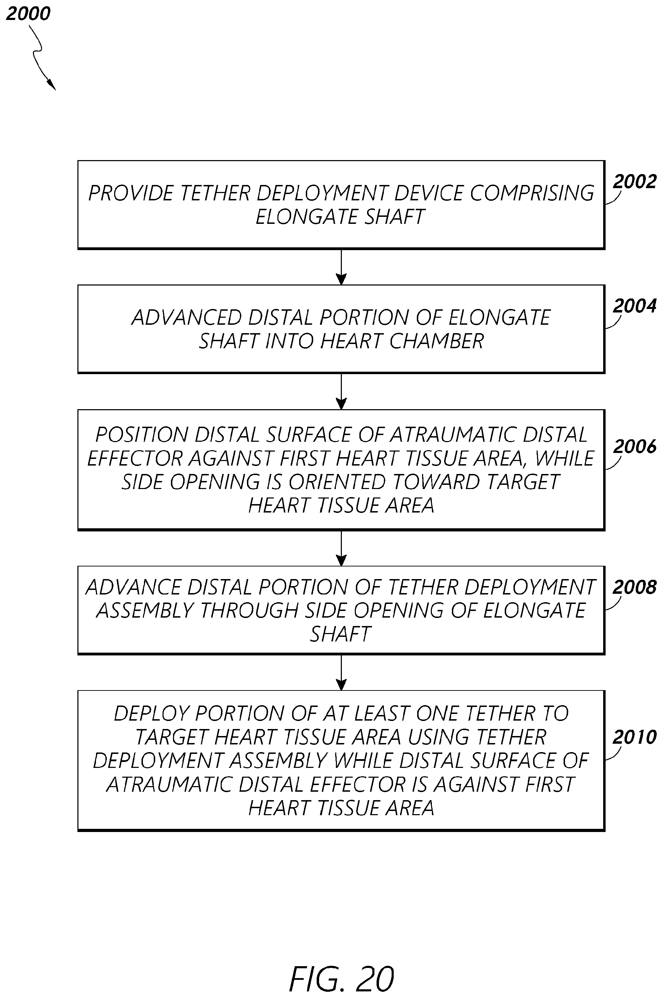

Figure 20 is a process flow diagram of an example of a process 2000 for deploying a tether using one or more tether deployment devices described herein. In block 2002, the process can involving providing a tether deployment device. The tether deployment device can have an elongate shaft comprising a lumen extending from a proximal end to a side opening on a distal portion thereof. The tether deployment device can comprise an atraumatic distal effector associated with, such as coupled to, the distal portion of the elongate shaft and disposed distally of the side opening. A tether deployment assembly can be configured to be at least partially received within the lumen of the elongate shaft. In block 2004, the process can involve advancing the distal portion of the elongate shaft into a heart chamber. In block 2006, the process can involve positioning a distally oriented surface of the atraumatic distal effector against a first tissue area, while the side opening is oriented toward a target tissue area. The first tissue area can be adjacent to the target tissue area. In block 2008, the process can involve advancing a distal portion of the tether deployment assembly through the side opening of the elongate shaft. In block 2010, the process can involve deploying a portion of at least one tether to the target tissue area using the tether deployment assembly while the distally oriented surface of the atraumatic distal effector is against the first tissue area.

In some instances, advancing the distal portion of the elongate shaft into a heart chamber can comprise advancing the distal portion of the elongate shaft into a heart ventricle. Positioning the distally oriented surface of the atraumatic distal effector against the first tissue area can comprise positioning the distally oriented surface against at least one of a portion of a heart annulus oriented toward the heart ventricle and a portion of heart tissue adjacent to the portion of the heart annulus. In some instances, deploying the portion of the at least one tether can comprise puncturing the heart annulus using a needle at a distal end of the tether deployment assembly, from a first surface of the heart annulus oriented toward the heart ventricle through to a second surface of the heart annulus oriented toward a heart atrium. In some instances, deploying the portion of the at least one tether can comprise deploying an anchor of the at least one tether over the second surface of the heart annulus oriented toward the atrium. Deploying the anchor can comprise forming a suture knot using the portion of the at least one tether. In some instances, deploying the portion of the at least one tether can comprise deploying a distal portion of a tether to a posterior portion of the heart annulus. The elongate shaft can be retracted back through the ventricle after the anchor is deployed to the target position of the heart annulus. Additional portions of the tether can be extended through the side opening of the elongate shaft as the elongate shaft is retracted. In some instances, deploying a proximal portion of the tether can comprise withdrawing the elongate shaft back through the heart ventricle and an opening formed in the anterior heart wall portion. For example, the elongate shaft can be withdrawn through the opening that was formed in the heart wall for accessing the ventricle. The proximal portion of the tether can be deployed to an anterior heart wall portion of the ventricle. For example, the posterior portion of the heart annulus can be extended through the opening and tethered to the anterior heart wall portion.

In some instances, advancing the distal portion of the elongate shaft into the heart chamber can comprise advancing the distal portion of the elongate shaft into a heart atrium. In some instances, positioning the distally oriented surface of the atraumatic distal effector against the first tissue area can comprise positioning the distally oriented surface against at least one of a portion of a heart annulus of a heart valve oriented toward the heart atrium and a portion of heart tissue adjacent to the portion of the heart annulus. Deploying the portion of the at least one tether can comprise puncturing the heart annulus using a needle at a distal end of the tether deployment assembly, from a first surface of the heart annulus oriented toward the heart atrium through to a second surface of the heart annulus oriented toward a heart ventricle. In some instances, deploying the portion of the at least one tether can comprise deploying an anchor of the at least one tether over the second surface of the heart annulus oriented toward the ventricle. For example, deploying the anchor can comprise forming a suture knot using the portion of the at least one tether.

In some instances, deploying the portion of the at least one tether can comprise deploying a distal portion of a first tether to an anterior portion of the heart annulus and a distal portion of a second tether to a posterior portion of the heart annulus. The first tether can be coupled to the second tether over atrium oriented surface portions of the heart valve. In some instances, coupling the first tether to the second tether can comprise forming a tie over the posterior portion of the heart annulus. In some instances, coupling the first tether to the second tether can comprise forming a tie over the anterior portion of the heart annulus. In some instances, coupling the first tether to the second tether can comprise forming a tie over a leaflet coaptation zone of the heart valve.

In some instances, deploying the distal portion of the first tether to the anterior portion of the heart annulus can comprise deploying the distal portion of the first tether to an anterior portion of a mitral valve heart annulus. Deploying the distal portion of the second tether to the posterior portion of the heart annulus can comprise deploying the distal portion of the second tether to a posterior portion of the mitral valve heart annulus.

In some instances, deploying the distal portion of the first tether to the anterior portion of a mitral valve heart annulus can comprise deploying the distal portion of the first tether to a portion of the mitral valve heart annulus adjacent to an anterolateral portion of an anterior leaflet. Deploying the distal portion of the second tether to the posterior portion of the mitral valve heart annulus can comprise deploying the distal portion of the second tether to a portion of the mitral valve heart annulus adjacent to a posterolateral scallop of a posterior leaflet. In some instances, deploying the distal portion of the first tether to the anterior portion of a mitral valve heart annulus can comprise deploying the distal portion of the first tether to a portion of the mitral valve heart annulus adjacent to an anteromiddle segment of an anterior leaflet. Deploying the distal portion of the second tether to the posterior portion of the mitral valve heart annulus can comprise deploying the distal portion of the second tether to a portion of the mitral valve heart annulus adjacent to a posteromiddle scallop of a posterior leaflet. In some instances, deploying the distal portion of the first tether to the anterior portion of a mitral valve heart annulus can comprise deploying the distal portion of the first tether to a portion of the mitral valve heart annulus adjacent to an anteromedial portion of an anterior leaflet. Deploying the distal portion of the second tether to the posterior portion of the mitral valve heart annulus can comprise deploying the distal portion of the second tether to a portion of the mitral valve heart annulus adjacent to a posteromedial scallop of a posterior leaflet. In some instances, the distal portions of the first and second tethers can be deployed to opposing portions of the annulus.

Conditional language used herein, such as, among others, "can," "could," "might," "may," *"e.g.,"* and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an openended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, *etc.* may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the disclosure and claims below should not be limited by the particular examples described above, but should be determined only by a fair reading of the claims that follow.

It should be understood that certain ordinal terms (*e.g.,* "first" or "second") may be provided for ease of reference and do not necessarily imply physical characteristics or ordering. Therefore, as used herein, an ordinal term (*e.g.,* "first," "second," "third," *etc.*) used to modify an element, such as a structure, a component, an operation, *etc.,* does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A tether deployment device shaft (402; 602; 702; 802; 902; 1102), comprising:
an elongate portion comprising a lumen (420; 620; 720; 920; 1120) configured to receive at least a portion of a tether deployment assembly (460; 660; 760; 860; 1160) configured to deploy a tether (480; 680; 880; 1180) to a target tissue area, the lumen (420; 620; 720; 920; 1120) extending from a proximal end of the elongate portion to a side opening (412; 612; 712; 812; 1112) on a distal portion thereof, an inner wall (430; 630; 730; 1130) defining the lumen (420; 620; 720; 920; 1120) comprising a curved wall portion (636; 736; 1136) extending from a longitudinal wall portion (632; 732; 1132) to the side opening (412; 612; 712; 812; 1112) to provide a curved path configured to guide an exit trajectory of a portion of the tether deployment assembly (460; 660; 760; 860; 1160) through the side opening (412; 612; 712; 812; 1112); and
an atraumatic distal effector (440; 640; 840; 940; 1140) coupled to the distal portion of the elongate portion distal of the side opening (412; 612; 712; 812; 912; 1112), **characterised in that** the longitudinal wall portion (632; 732; 1132) extends from the proximal end (408; 608; 1108) of the elongate portion to a proximal end (414; 614; 714; 1114) of the side opening (412; 612; 712; 812; 1112) along an axis parallel or substantially parallel to a longitudinal axis of the elongate portion, and the curved wall portion (636; 736; 1136) extends from the longitudinal wall portion to a distal end of the side opening (412; 612; 712; 812; 912; 1112).

2. The shaft (602) of claim 1, wherein the inner wall (630) comprising the curved wall portion comprises a groove (638) on at least a portion, a portion of the tether deployment assembly being configured to contact at least a portion of the groove (638) while being advanced out of the lumen (620) to guide the exit of the tether deployment assembly, preferably, wherein the groove (630) is on a portion of the inner wall opposite the side opening (612).

3. The shaft (902) of any one of claims 1 to 2, wherein the atraumatic distal effector (940) comprises an inflatable balloon (942) positioned at least partially around the distal portion of the elongate portion (902), a distal end of the inflatable balloon (942), while the inflatable balloon (942) is in the inflated state, being distal of a distal end of the elongate portion and being configured to contact a first tissue area adjacent to the target tissue area.

4. The shaft (402; 602; 702; 802; 902; 1102) of any one of claims 1 to 2, wherein the atraumatic distal effector (440; 640; 840; 940; 1140) comprises a flexible disc coupled to a distal end (410; 610; 810; 1110) of the elongate portion.

5. The shaft (402; 602; 702; 802; 1102) of claim 4, wherein the flexible disc is perpendicularly oriented relative to a longitudinal axis of the elongate portion, or, wherein a portion of the elongate portion distal of the side opening (412; 612; 712; 812; 1112) is flexible and an orientation of the flexible disc is rotatable relative to the elongate portion about an axis perpendicular to a longitudinal axis of the elongate portion.

6. A tether deployment device (400; 600; 700; 800; 900; 1100), comprising:
a tether deployment assembly (460; 660; 760; 860; 1160) comprising a tether (480; 680; 880; 1180), the tether comprising a distal portion (484; 684; 1184) configured to be deployed onto a target tissue area;
a tether deployment device shaft (402; 602; 702; 802; 902; 1102) according to claim 1.

7. The device (600) of claim 6, wherein the inner wall (630) comprising the curved wall portion (636) comprises a groove (638) on at least a portion, a portion of the tether deployment assembly (660) being configured to contact at least a portion of the groove (638) while being advanced out of the lumen (620) to guide the exit of the tether deployment assembly (660), preferably, wherein the groove (638) is on a portion of the inner wall (630) opposite the side opening (612).

8. The device (900) of any one of claims 6 to 7, wherein the atraumatic distal effector (940) comprises an inflatable balloon (942) positioned at least partially around the distal portion (906) of the elongate portion (902), a distal end (952) of the inflatable balloon (942), while the inflatable balloon (942) is in an inflated state, being distal of a distal end (910) of the elongate portion (902) and being configured to contact a first tissue area adjacent to the target tissue area.

9. The device (400; 600; 700; 800; 1100) of any one of claims 6 to 7, wherein the atraumatic distal effector (440; 640; 840; 1140) comprises a flexible disc coupled to a distal end (410; 610; 810; 1110) of the elongate portion (402; 602; 702; 802; 1102).

10. The device (400; 600; 700; 800; 1100) of claim 9, wherein the flexible disc is perpendicularly oriented relative to a longitudinal axis of the elongate portion (402; 602; 702; 802; 1102).

11. The device of claim 10, wherein a portion of the distal portion of the elongate portion (402; 602; 702; 802; 1102) distal of the side opening (412; 612; 712; 812; 912; 1112) is flexible and an orientation of the flexible disc is rotatable relative to the elongate portion (402; 602; 702; 802; 1102) about an axis perpendicular to a longitudinal axis of the elongate portion (402; 602; 702; 802; 1102).

12. The device (400; 1100) of any one of claims 6 to 7, wherein a proximal portion (1104) of the elongate portion (402; 1102) is configured to be coupled to a handle (500), the handle (500) comprising a lateral portion (520) extending laterally of a longitudinal axis of the elongate portion (402; 1102) and configured to engage with an operator, the side opening (412; 1112) being on a portion of the elongate portion (402; 1102) having the opposite orientation as the lateral portion (520) of the handle (500) or the same as that of the lateral portion (520) of the handle (500).

13. The device (400; 1100) of any one of claims 6 to 7, wherein the tether deployment assembly (460; 1160) comprises a needle (470; 1170) at a distal end (468; 1168) configured to puncture the target tissue area to deploy the distal portion (484; 1184) of the tether (480; 1180) to the target tissue area.

14. The device (400; 1100) of any one of claims 6 to 7, wherein the tether deployment assembly (460; 1160) is configured to form a suture knot over a surface of the target tissue area using the distal portion (484; 1184) of the tether (480; 1180).

15. A method of simulating tethering, comprising:
providing a tether deployment device (400; 600; 700; 800; 900; 1100) as defined in any of claims 6 to 14;
advancing the distal portion of the elongate portion (402; 602; 702; 802; 902; 1102) into a simulated heart chamber;
positioning a distally oriented surface of the atraumatic distal effector (440; 640; 840; 940; 1140) against a first simulated tissue area, while the side opening (412; 612; 712; 812; 912; 1112) is oriented toward a target simulated tissue area;
advancing a distal portion of the tether deployment assembly (460; 660; 760; 860; 1160) through the side opening (412; 612; 712; 812; 912; 1112) of the elongate portion (402; 602; 702; 802; 902; 1102); and
deploying a portion of at least one tether (480; 680; 880; 1180) to the target simulated tissue area using the tether deployment assembly (460; 660; 760; 860; 1160) while the distally oriented surface of the atraumatic distal effector (440; 640; 840; 940; 1140) is against the first simulated tissue area.

## Patentansprüche

1. Leinenausbringvorrichtungsschaft (402; 602; 702; 802; 902; 1102), umfassend:
einen langgestreckten Abschnitt, der ein Lumen (420; 620; 720; 920; 1120) umfasst, das dazu ausgelegt ist, zumindest einen Teil einer Leinenausbringanordnung (460; 660; 760; 860; 1160) aufzunehmen, die dazu ausgelegt ist, eine Leine (480; 680; 880; 1180) in einen Zielgewebebereich auszubringen, wobei sich das Lumen (420; 620; 720; 920; 1120) von einem proximalen Ende des langgestreckten Abschnitts bis zu einer seitlichen Öffnung (412; 612; 712; 812; 1112) an einem distalen Abschnitt desselben erstreckt, wobei eine Innenwand (430; 630; 730; 1130), die das Lumen (420; 620; 720; 920; 1120) definiert, die einen gekrümmten Wandabschnitt (636; 736; 1136) umfasst, der sich von einem Längswandabschnitt (632; 732; 1132) bis zur seitlichen Öffnung (412; 612; 712; 812; 1112) erstreckt, um einen gekrümmten Pfad bereitzustellen, der dazu konfiguriert ist, eine Austrittsbahn eines Abschnitts der Leinenausbringanordnung (460; 660; 760; 860; 1160) durch die seitliche Öffnung (412; 612; 712; 812; 1112) zu führen; und
einen atraumatischen distalen Effektor (440; 640; 840; 940; 1140), der mit dem distalen Abschnitt des langgestreckten Abschnitts distal der seitlichen Öffnung (412; 612; 712; 812; 912; 1112) gekoppelt ist, **dadurch gekennzeichnet, dass** sich der Längswandabschnitt (632; 732; 1132) von dem proximalen Ende (408; 608; 1108) des langgestreckten Abschnitts bis zu einem proximalen Ende (414; 614; 714; 1114) der seitlichen Öffnung (412; 612; 712; 812; 1112) entlang einer Achse, die parallel oder im Wesentlichen parallel zu einer Längsachse des langgestreckten Abschnitts verläuft, erstreckt und sich der gekrümmte Wandabschnitt (636; 736; 1136) vom Längswandabschnitt zu einem distalen Ende der seitlichen Öffnung (412; 612; 712; 812; 912; 1112) erstreckt.

2. Schaft (602) nach Anspruch 1, wobei die Innenwand (630), die den gekrümmten Wandabschnitt umfasst, an mindestens einem Abschnitt eine Nut (638) aufweist, wobei ein Abschnitt der Leinenausbringanordnung dazu ausgelegt ist, mindestens einen Abschnitt der Nut (638) zu berühren, während sie aus dem Lumen (620) vorgeschoben wird, um den Austritt der Leinenausbringanordnung zu führen, wobei vorzugsweise die Nut (630) an einem Abschnitt der Innenwand gegenüber der seitlichen Öffnung (612) angeordnet ist.

3. Schaft (902) nach einem der Ansprüche 1 bis 2, wobei der atraumatische distale Effektor (940) einen aufpumpbaren Ballon (942) umfasst, der zumindest teilweise um den distalen Abschnitt des langgestreckten Abschnitts (902) herum angeordnet ist, wobei ein distales Ende des aufpumpbaren Ballons (942), während sich der aufpumpbare Ballon (942) im aufgepumpten Zustand befindet, distal zu einem distalen Ende des langgestreckten Abschnitts liegt und dazu ausgelegt ist, einen ersten Gewebebereich zu berühren, der zu dem Zielgewebebereich benachbart ist.

4. Schaft (402; 602; 702; 802; 902; 1102) nach einem der Ansprüche 1 bis 2, wobei der atraumatische distale Effektor (440; 640; 840; 940; 1140) eine flexible Scheibe umfasst, die mit einem distalen Ende (410; 610; 810; 1110) des langgestreckten Abschnitts gekoppelt ist.

5. Schaft (402; 602; 702; 802; 1102) nach Anspruch 4, wobei die flexible Scheibe senkrecht zu einer Längsachse des langgestreckten Abschnitts ausgerichtet ist, oder wobei ein Abschnitt des langgestreckten Abschnitts distal von der seitlichen Öffnung (412; 612; 712; 812; 1112) flexibel ist und eine Ausrichtung der flexiblen Scheibe relativ zum langgestreckten Abschnitt um eine Achse drehbar ist, die senkrecht zu einer Längsachse des langgestreckten Abschnitts verläuft.

6. Leinenausbringvorrichtung (400; 600; 700; 800; 900; 1100), umfassend:
eine Leinenausbringanordnung (460; 660; 760; 860; 1160), die eine Leine (480; 680; 880; 1180) umfasst, wobei die Leine einen distalen Abschnitt (484; 684; 1184) aufweist, der dazu ausgelegt ist, auf einem Zielgewebebereich ausgebracht zu werden;
einen Leinenausbringvorrichtungsschaft (402; 602; 702; 802; 902; 1102) nach Anspruch 1.

7. Vorrichtung (600) nach Anspruch 6, wobei die den gekrümmten Wandabschnitt (636) umfassende Innenwand (630) an mindestens einem Abschnitt eine Nut (638) umfasst, wobei ein Abschnitt der Leinenausbringvorrichtung (660) dazu ausgelegt ist, mindestens einen Abschnitt der Nut zu berühren (638) zu berühren, während sie aus dem Lumen (620) vorgeschoben wird, um den Austritt der Leinenausbringvorrichtung (660) zu führen, wobei vorzugsweise die Nut (638) an einem Abschnitt der Innenwand (630) gegenüber der Seitenöffnung (612) angeordnet ist.

8. Vorrichtung (900) nach einem der Ansprüche 6 bis 7, wobei der atraumatische distale Effektor (940) einen aufpumpbaren Ballon (942) umfasst, der zumindest teilweise um den distalen Abschnitt (906) des langgestreckten Abschnitts (902) herum angeordnet ist, wobei ein distales Ende (952) des aufpumpbaren Ballons (942), während sich der aufpumpbare Ballon (942) in einem aufgepumpten Zustand befindet, distal zu einem distalen Ende (910) des langgestreckten Abschnitts (902) liegt und dazu ausgelegt ist, einen ersten Gewebebereich zu berühren, der zu dem Zielgewebebereich benachbart ist.

9. Vorrichtung (400; 600; 700; 800; 1100) nach einem der Ansprüche 6 bis 7, wobei der atraumatische distale Effektor (440; 640; 840; 1140) eine flexible Scheibe umfasst, die mit einem distalen Ende (410; 610; 810; 1110) des langgestreckten Abschnitts (402; 602; 702; 802; 1102) gekoppelt ist.

10. Vorrichtung (400; 600; 700; 800; 1100) nach Anspruch 9, wobei die flexible Scheibe senkrecht zu einer Längsachse des langgestreckten Abschnitts (402; 602; 702; 802; 1102) ausgerichtet ist.

11. Vorrichtung nach Anspruch 10, wobei ein Abschnitt des distalen Teils des langgestreckten Abschnitts (402; 602; 702; 802; 1102) distal von der seitlichen Öffnung (412; 612; 712; 812; 912; 1112) flexibel ist und eine Ausrichtung der flexiblen Scheibe relativ zum langgestreckten Abschnitt (402; 602; 702; 802; 1102) um eine Achse drehbar ist, die senkrecht zu einer Längsachse des langgestreckten Abschnitts (402; 602; 702; 802; 1102) verläuft.

12. Vorrichtung (400; 1100) nach einem der Ansprüche 6 bis 7, wobei ein proximaler Abschnitt (1104) des langgestreckten Abschnitts (402; 1102) dazu ausgelegt ist, mit einem Griff (500) gekoppelt zu werden, wobei der Griff (500) einen seitlichen Abschnitt (520) umfasst, der sich seitlich einer Längsachse des langgestreckten Abschnitts (402; 1102) erstreckt und dazu ausgelegt ist, mit einem Bediener zusammenzuwirken, wobei sich die seitliche Öffnung (412; 1112) an einem Abschnitt des langgestreckten Abschnitts (402; 1102) befindet, der eine entgegengesetzte Ausrichtung wie der seitliche Abschnitt (520) des Griffs (500) aufweist oder die gleiche Ausrichtung wie der seitliche Abschnitt (520) des Griffs (500) aufweist.

13. Vorrichtung (400; 1100) nach einem der Ansprüche 6 bis 7, wobei die Leinenausbringanordnung (460; 1160) eine Nadel (470; 1170) an einem distalen Ende (468; 1168) aufweist, die dazu ausgelegt ist, den Zielgewebebereich zu punktieren, um den distalen Abschnitt (484; 1184) der Leine (480; 1180) in dem Zielgewebebereich auszubringen.

14. Vorrichtung (400; 1100) nach einem der Ansprüche 6 bis 7, wobei die Leinenausbringanordnung (460; 1160) dazu ausgelegt ist, unter Verwendung des distalen Abschnitts (484; 1184) der Leine (480; 1180) einen Nahtknoten über einer Oberfläche des Zielgewebebereichs zu bilden.

15. Verfahren zur Simulation einer Leinenanbindung, umfassend:
Bereitstellung einer Leinenausbringvorrichtung (400; 600; 700; 800; 900; 1100), wie in einem der Ansprüche 6 bis 14 definiert;
Vorschieben des distalen Abschnitts des langgestreckten Abschnitts (402; 602; 702; 802; 902; 1102) in eine simulierte Herzkammer;
Anlegen einer distal ausgerichteten Oberfläche des atraumatischen distalen Effektors (440; 640; 840; 940; 1140) an einen ersten simulierten Gewebebereich, während die seitliche Öffnung (412; 612; 712; 812; 912; 1112) in Richtung zu einem simulierten Zielgewebebereich ausgerichtet ist;
Vorschieben eines distalen Abschnitts der Leinenausbringanordnung (460; 660; 760; 860; 1160) durch die seitliche Öffnung (412; 612; 712; 812; 912; 1112) des langgestreckten Abschnitts (402; 602; 702; 802; 902; 1102); und
Ausbringen eines Abschnitts mindestens einer Leine (480; 680; 880; 1180) in den simulierten Zielgewebebereich unter Verwendung der Leinenausbringanordnung (460; 660; 760; 860; 1160), während die distal ausgerichtete Oberfläche des atraumatischen distalen Effektors (440; 640; 840; 940; 1140) an dem ersten simulierten Gewebebereich anliegt.

## Revendications

1. Tige (402 ; 602 ; 702 ; 802 ; 902 ; 1102) de dispositif de déploiement d'attache, comprenant :
une partie allongée comprenant une lumière (420 ; 620 ; 720 ; 920 ; 1120) conçue pour recevoir au moins une partie d'un ensemble de déploiement (460 ; 660 ; 760 ; 860 ; 1160) d'attache conçu pour déployer une attache (480 ; 680 ; 880 ; 1180) au niveau d'une zone tissulaire cible, la lumière (420 ; 620 ; 720 ; 920 ; 1120) s'étendant depuis une extrémité proximale de la partie allongée à une ouverture latérale (412 ; 612 ; 712 ; 812 ; 1112) sur une partie distale correspondante, une paroi interne (430 ; 630 ; 730 ; 1130) définissant la lumière (420 ; 620 ; 720 ; 920 ; 1120) comprenant une partie paroi incurvée (636 ; 736 ; 1136) s'étendant depuis une partie paroi longitudinale (632 ; 732 ; 1132) à l'ouverture latérale (412 ; 612 ; 712 ; 812 ; 1112) afin de fournir un trajet incurvé conçu pour guider une trajectoire de sortie d'une partie de l'ensemble de déploiement (460 ; 660 ; 760 ; 860 ; 1160) d'attache à travers l'ouverture latérale (412 ; 612 ; 712 ; 812 ; 1112) ; et
un effecteur distal atraumatique (440 ; 640 ; 840 ; 940 ; 1140) accouplé à la partie distale de la partie allongée en position distale par rapport à l'ouverture latérale (412 ; 612 ; 712 ; 812 ; 912 ; 1112), **caractérisée en ce que** la partie paroi longitudinale (632 ; 732 ; 1132) s'étend depuis l'extrémité proximale (408 ; 608 ; 1108) de la partie allongée à une extrémité proximale (414 ; 614 ; 714 ; 1114) de l'ouverture latérale (412 ; 612 ; 712 ; 812 ; 1112) le long d'un axe parallèle ou sensiblement parallèle à un axe longitudinal de la partie allongée et la partie paroi incurvée (636 ; 736 ; 1136) s'étend depuis la partie paroi longitudinale à une extrémité distale de l'ouverture latérale (412 ; 612 ; 712 ; 812 ; 912 ; 1112).

2. Tige (602) selon la revendication 1, la paroi interne (630) comprenant la partie paroi incurvée comprenant une rainure (638) située sur au moins une partie, une partie de l'ensemble de déploiement d'attache étant conçue pour entrer en contact avec au moins une partie de la rainure (638) tout en étant avancée hors de la lumière (620) afin de guider la sortie de l'ensemble de déploiement d'attache, de préférence, la rainure (630 ) étant située sur une partie de la paroi interne opposée à l'ouverture latérale (612).

3. Tige (902) selon l'une quelconque des revendications 1 à 2, l'effecteur distal atraumatique (940) comprenant un ballonnet gonflable (942) positionné au moins partiellement autour de la partie distale de la partie allongée (902), une extrémité distale du ballonnet gonflable (942), tandis que le ballonnet gonflable (942) est dans un état gonflé, étant en position distale par rapport à une extrémité distale de la partie allongée et étant conçue pour entrer en contact avec une première zone tissulaire adjacente à la zone tissulaire cible.

4. Tige (402 ; 602 ; 702 ; 802 ; 902 ; 1102) selon l'une quelconque des revendications 1 à 2, l'effecteur distal atraumatique (440 ; 640 ; 840 ; 940 ; 1140) comprenant un disque flexible accouplé à une extrémité distale (410 ; 610 ; 810 ; 1110) de la partie allongée.

5. Tige (402 ; 602 ; 702 ; 802 ; 1102) selon la revendication 4, le disque flexible étant orienté perpendiculairement par rapport à un axe longitudinal de la partie allongée ou une partie de la partie allongée en position distale par rapport à l'ouverture latérale (412 ; 612 ; 712 ; 812 ; 1112) étant flexible et une orientation du disque flexible pouvant tourner par rapport à la partie allongée autour d'un axe perpendiculaire à un axe longitudinal de la partie allongée.

6. Dispositif de déploiement (400 ; 600 ; 700 ; 800 ; 900 ; 1100) d'attache, comprenant :
un ensemble de déploiement (460 ; 660 ; 760 ; 860 ; 1160) d'attache comprenant une attache (480 ; 680 ; 880 ; 1180), l'attache comprenant une partie distale (484 ; 684 ; 1184) conçue pour être déployée sur une zone tissulaire cible ;
une tige (402 ; 602 ; 702 ; 802 ; 902 ; 1102) de dispositif de déploiement d'attache selon la revendication 1.

7. Dispositif (600) selon la revendication 6, la paroi interne (630) comprenant la partie paroi incurvée (636) comprenant une rainure (638) située sur au moins une partie, une partie de l'ensemble de déploiement (660) d'attache étant conçue pour entrer en contact avec au moins une partie de la rainure (638) tout en étant avancée hors de la lumière (620) afin de guider la sortie de l'ensemble de déploiement (660) d'attache, de préférence, la rainure (638) étant située sur une partie de la paroi interne (630) opposée à l'ouverture latérale (612).

8. Dispositif (900) selon l'une quelconque des revendications 6 à 7, l'effecteur distal atraumatique (940) comprenant un ballonnet gonflable (942) positionné au moins partiellement autour de la partie distale (906) de la partie allongée (902), une extrémité distale (952) du ballonnet gonflable (942), tandis que le ballonnet gonflable (942) est dans un état gonflé, étant en position distale par rapport à une extrémité distale (910) de la partie allongée (902) et étant conçue pour entrer en contact avec une première zone tissulaire adjacente à la zone tissulaire cible.

9. Dispositif (400 ; 600 ; 700 ; 800 ; 1100) selon l'une quelconque des revendications 6 à 7, l'effecteur distal atraumatique (440 ; 640 ; 840 ; 1140) comprenant un disque flexible accouplé à une extrémité distale (410 ; 610 ; 810 ; 1110) de la partie allongée (402 ; 602 ; 702 ; 802 ; 1102).

10. Dispositif (400 ; 600 ; 700 ; 800 ; 1100) selon la revendication 9, le disque flexible étant orienté perpendiculairement par rapport à un axe longitudinal de la partie allongée (402 ; 602 ; 702 ; 802 ; 1102).

11. Dispositif selon la revendication 10, une partie de la partie distale de la partie allongée (402 ; 602 ; 702 ; 802 ; 1102) en position distale par rapport à l'ouverture latérale (412 ; 612 ; 712 ; 812 ; 912 ; 1112) étant flexible et une orientation du disque flexible pouvant tourner par rapport à la partie allongée (402 ; 602 ; 702 ; 802 ; 1102) autour d'un axe perpendiculaire à un axe longitudinal de la partie allongée (402 ; 602 ; 702 ; 802 ; 1102).

12. Dispositif (400 ; 1100) selon l'une quelconque des revendications 6 à 7, une partie proximale (1104) de la partie allongée (402 ; 1102) étant conçue pour être accouplée à une poignée (500), la poignée (500) comprenant une partie latérale (520) s'étendant latéralement par rapport à un axe longitudinal de la partie allongée (402 ; 1102) et étant conçue pour venir en prise avec un opérateur, l'ouverture latérale (412 ; 1112) située sur une partie de la partie allongée (402 ; 1102) présentant l'orientation opposée à celle de la partie latérale (520) de la poignée (500) ou la même que celle de la partie latérale (520) de la poignée (500).

13. Dispositif (400 ; 1100) selon l'une quelconque des revendications 6 à 7, l'ensemble de déploiement (460 ; 1160) d'attache comprenant une aiguille (470 ; 1170) au niveau d'une extrémité distale (468 ; 1168) conçue pour percer la zone tissulaire cible afin de déployer la partie distale (484 ; 1184) de l'attache (480 ; 1180) au niveau de la zone tissulaire cible.

14. Dispositif (400 ; 1100) selon l'une quelconque des revendications 6 à 7, l'ensemble de déploiement (460 ; 1160) d'attache étant conçu pour former un noeud de suture sur une surface de la zone tissulaire cible à l'aide de la partie distale (484 ; 1184) de l'attache (480 ; 1180).

15. Procédé de simulation d'attache, comprenant :
la fourniture d'un dispositif de déploiement (400 ; 600 ; 700 ; 800 ; 900 ; 1100) d'attache tel que défini dans l'une quelconque des revendications 6 à 14 ;
le fait de faire avancer la partie distale de la partie allongée (402 ; 602 ; 702 ; 802 ; 902 ; 1102) dans une chambre cardiaque simulée ;
le positionnement d'une surface orientée distalement de l'effecteur distal atraumatique (440 ; 640 ; 840 ; 940 ; 1140) contre une première zone tissulaire simulée, tandis que l'ouverture latérale (412 ; 612 ; 712 ; 812 ; 912 ; 1112) est orientée vers une zone tissulaire simulée cible ;
le fait de faire avancer une partie distale de l'ensemble de déploiement (460 ; 660 ; 760 ; 860 ; 1160) d'attache à travers l'ouverture latérale (412 ; 612 ; 712 ; 812 ; 912 ; 1112) de la partie allongée (402 ; 602 ; 702 ; 802 ; 902 ; 1102) ; et
le déploiement d'une partie d'au moins une attache (480 ; 680 ; 880 ; 1180) au niveau de la zone tissulaire simulée cible à l'aide de l'ensemble de déploiement (460 ; 660 ; 760 ; 860 ; 1160) d'attache tandis que la surface orientée distalement de l'effecteur distal atraumatique (440 ; 640 ; 840 ; 940 ; 1140) est contre la première zone tissulaire simulée.
